# EUROPEAN PATENT APPLICATION

(11) **EP 2 911 079 A2**
(43) Date of publication of application: **26.08.2015**
(21) Application number: 15156004.2
(22) Date of filing: 20.02.2015
(51) Int. Cl.: G06F 21/50

(54) **Healthcare fraud sharing system**

(30) Priority: 20.02.2014 US 201461942480 P; 30.04.2014 US 201461986783 P; 29.05.2014 US 201462004651 P; 20.10.2014 US 201414518757
(71) Applicant: Palantir Technologies, Inc., Palo Alto, California 94301 (US)
(72) Inventor: Albertson, Jacob, Palo Alto, CA 94301 (US); Wang, Lekan, Palo Alto, CA 94301 (US); Cox, Taylor, Palo Alto, CA 94301 (US); Burchhardt, Chris, Palo Alto, CA 94301 (US); Ketterling, Casey, Palo Alto, CA 94301 (US); Sudan, Ajay, Palo Alto, CA 94301 (US); McGrew, Bob, Palo Alto, CA 94301 (US); Hildebrandt, Melody, Palo Alto, CA 94301 (US); Sing, Harkirat, Palo Alto, CA 94301 (US); Sankar, Shyam, Palo Alto, CA 94301 (US); Ducott, Rick, Palo Alto, CA 94301 (US); Maag, Peter, Palo Alto, CA 94301 (US); Kimball, Marissa, Palo Alto, CA 94301 (US)
(74) Representative: Potter, Julian Mark

(57) **Abstract**

Systems and techniques for sharing healthcare fraud data are described herein. Healthcare fraud detection schemes and/or fraud data may be automatically shared, investigated, enabled, and/or used by entities. A healthcare fraud detection scheme may be enabled on different entities comprising different computing systems to combat similar healthcare fraud threats, instances, and/or attacks. Healthcare fraud detection schemes and/or fraud data may be modified to redact sensitive information and/or configured through access controls for sharing.

## Description

### INCORPORATION BY REFERENCE TO ANY PRIORITY APPLICATIONS

Any and all applications for which a foreign or domestic priority claim is identified in the Application Data Sheet as filed with the present application are hereby incorporated by reference under 37 CFR 1.57.

This application claims benefit of U.S. Provisional Patent Application Serial No. 61/942,480 entitled "Security Sharing System" filed February 20, 2014, which is hereby incorporated by reference in its entirety.

This application claims benefit of U.S. Provisional Patent Application Serial No. 61/986,783 entitled "Healthcare Fraud Sharing System" filed April 30, 2014, which is hereby incorporated by reference in its entirety.

This application claims benefit of U.S. Provisional Patent Application Serial No. 62/004,651 entitled "Healthcare Fraud Sharing System" filed May 29, 2014, which is hereby incorporated by reference in its entirety.

### CROSS-REFERENCE TO RELATED APPLICATIONS

This application is related to but does not claim priority from U.S. Patent Application Serial No. 13/968,265 entitled "Generating Data Clusters With Customizable Analysis Strategies" filed August 15, 2013, and U.S. Patent Application Serial No. 13/968,213 entitled "Prioritizing Data Clusters With Customizable Scoring Strategies" filed August 15, 2013, which are hereby incorporated by reference in their entireties and collectively referred to herein as the "Cluster references."

This application is related to but does not claim priority from U.S. Patent No. 8,515,912 entitled "Sharing And Deconflicting Data Changes In A Multimaster Database System" filed July 15, 2010, U.S. Patent No. 8,527,461 entitled "Cross-ACL Multi-Master Replication" filed November 27, 2012, U.S. Patent Application Serial No. 13/076,804 entitled "Cross-Ontology Multi-Master Replication" filed March 31, 2011, U.S. Patent Application Serial No. 13/657,684 entitled "Sharing Information Between Nexuses That Use Different Classification Schemes For Information Access Control" filed October 22, 2012, and U.S. Patent Application Serial No. 13/922,437 entitled "System And Method For Incrementally Replicating Investigative Analysis Data" filed June 20, 2013, which are hereby incorporated by reference in their entireties and collectively referred to herein as the "Sharing references."

This application is related to but does not claim priority from U.S. Patent No. 8,489,623 entitled "Creating Data In A Data Store Using A Dynamic Ontology" filed May 12, 2011, which is hereby incorporated by reference in its entirety and referred to herein as the "Ontology reference."

This application is related to but does not claim priority from U.S. Patent Application Serial No. 14/223,918 entitled "Verifiable Redactable Audit Log" filed March 24, 2014, which is hereby incorporated by reference in its entirety and referred to herein as the "Audit reference."

### BACKGROUND

In the area of computer-based platforms, healthcare fraud data may be collected, analyzed, and used to protect healthcare providers from fraud.

### SUMMARY

The systems, methods, and devices described herein each have several aspects, no single one of which is solely responsible for its desirable attributes. Without limiting the scope of this disclosure, several non-limiting features will now be discussed briefly.

In some embodiments, a system for sharing healthcare fraud information comprises one or more computing devices programmed, via executable code instructions. When executed, the executable code instructions may cause the system to implement a fraud data unit. The fraud data unit may be configured to receive a plurality of healthcare fraud data from one or more entities. The healthcare fraud data may comprise information regarding one or more healthcare fraud attacks detected by respective entities. When further executed, the executable code instructions may cause the system to implement a scheme unit. The scheme unit may be configured to receive a scheme from a first entity of a plurality of entities. A scheme may be based at least in part on healthcare fraud data associated with the first entity (e.g., detected by the first entity), wherein the scheme may be configured to be executable by one or more entities to recognize more healthcare fraud attacks. When further executed, the code instructions may cause the system to implement a fraud data modification unit. The fraud data modification unit may be configured to redact portions of the healthcare fraud data from various entities such that the redacted portions are not detectable in the healthcare fraud. The fraud data modification may be further configured to redact portions of the scheme such that the redacted portions are not detectable scheme. When further executed, the code instructions may cause the system to implement a distribution unit. The distribution unit may be configured to share the healthcare fraud data with multiple entities. The distribution unit any further configured to share the scheme with multiple entities.

In some embodiments, a non-transitory computer storage comprises instructions for causing one or more computing devices to share healthcare fraud information. When executed, the instructions may receive a plurality of healthcare related data from one or more open source data sources. When further executed, the instructions may receive a scheme from a first entity of a plurality of entities. The scheme may be configured to be executable by one or more entities to recognize one or more healthcare fraud attacks based at least in part on healthcare related data from at least one open source data source. When further executed, the instructions may transmit the scheme to one or more entities. The transmission of the scheme to one or more entities may be in accordance with sharing rules established by the one or more entities.

In some embodiments, a computer-implemented method for sharing healthcare fraud information comprises receiving a plurality of healthcare fraud data from one or more entities. The healthcare fraud data may comprise information regarding one or more healthcare fraud attacks detected by respective entities. The method may further comprise receiving a scheme. The scheme may be based at least in part on the plurality of healthcare fraud attack information from the one or more entities. The scheme may be configured to be executable by a plurality of the entities to recognize one or more healthcare fraud attacks. The method may further comprise transmitting the scheme to one or more entities.

### BRIEF DESCRIPTION OF THE DRAWINGS

Certain aspects of the disclosure will become more readily appreciated as those aspects become better understood by reference to the following detailed description, when taken in conjunction with the accompanying drawings.
Figure 1 is a block diagram illustrating an example healthcare fraud sharing system, according to some embodiments of the present disclosure.
Figure 2 is a flowchart illustrating an example scheme generating and sharing process, according to some embodiments of the present disclosure.
Figure 3 is a flowchart illustrating an example fraud data sharing process, according to some embodiments of the present disclosure.
Figure 4 is a flowchart illustrating an example modification process for fraud data and/or schemes, according to some embodiments of the present disclosure.
Figure 5 is a block diagram illustrating an example healthcare fraud sharing system sharing fraud data, schemes, and/or modified fraud data, according to some embodiments of the present disclosure.
Figure 6 is a flowchart illustrating an example scheme sharing process for multiple fraud attacks, according to some embodiments of the present disclosure.
Figure 7 is a block diagram illustrating an example healthcare fraud sharing system sharing fraud data, schemes, and/or modified fraud data from different entities, according to some embodiments of the present disclosure.
Figure 8A illustrates an example healthcare fraud sharing and/or redaction table, according to some embodiments of the present disclosure.
Figure 8B illustrates example fraud data and/or modified fraud data based on healthcare fraud attacks, according to some embodiments of the present disclosure.
Figure 8C illustrates example schemes in a format comprising code instructions, according to some embodiments of the present disclosure.
Figure 9A illustrates an example user interface of the healthcare fraud sharing system, according to some embodiments of the present disclosure.
Figure 9B illustrates an example user interface for viewing data objects within the healthcare fraud sharing system, according to some embodiments of the present disclosure.
Figure 10 is a block diagram illustrating an example healthcare fraud sharing system with which various methods and systems discussed herein may be implemented.

### DETAILED DESCRIPTION

Healthcare fraud information may be shared with the goal of improving particular aspects of healthcare fraud detection. For example, in a computer-based context, healthcare fraud information and strategies may be orally shared among institutions and/or individuals at a healthcare fraud conference. Additionally, insider tips may be shared from person to person to exchange healthcare fraud information. The institutions and/or individuals may then implement detection and/or defensive strategies through their computing systems with rules-based filters or retrospective claims investigations. The traditional patchwork of fraud detection often fails against sophisticated, adaptive, and determined adversaries.

In addition to sharing of healthcare fraud data orally, disclosed herein are systems for the secure sharing of healthcare fraud information and/or detection strategies among multiple entities. Using the techniques and systems described herein, healthcare fraud threats and/or healthcare waste may be addressed in near or real time more preemptively and/or efficiently by utilizing more information and/or analysis from other entities. Those techniques and systems may comprise automatic and/or in an ad hoc manner sharing fraud information and/or generic schemes to combat healthcare fraud threats and/or waste.

Sharing attack, fraud, and/or waste information may allow for distributive and/or efficient responses to healthcare fraud threats and/or waste. Institutions, organizations, entities, and/or the government may share attack and/or waste information automatically and/or in an ad hoc manner. The healthcare fraud sharing system may modify fraud and/or waste data to redact confidential, personal, and/or sensitive information for sharing with other entities.

Driven by the prospect of financial gain via fraud or abuse, fraud perpetrators attack from multiple angles using an ever expanding set of fraud schemes. In contrast, organizations often operate in silos and are unable to singlehandedly detect and mitigate the sheer volume and diversity of health fraud.

The healthcare fraud sharing system may enable participating organizations to exchange critical information and/or context about emerging health fraud threats in real time, subject to highly granular access controls and/or automatic redaction of sensitive data. In some embodiments, secure sharing, which may be achieved through secure communication protocols (e.g., one or more encryption standards and/or protocols), access controls, and/or the redaction of data, may enable compliancy with laws governing the handling of personal and/or healthcare-related data. For example, the Health Insurance Portability and Accountability Act ("HIPAA") may mandate that certain information be redacted and/or de-identified. Participating organizations may instantly gain access to real or near time feeds and/or intelligence, shared and enriched by participants across multiple industries and/or geographic boundaries. With the healthcare fraud sharing system, participating organizations may collaboratively improve situational awareness, obtain a comprehensive understanding of threats facing their organizations, and/or harden collective defenses against a wide range of fraud threats. In some embodiments, the healthcare fraud sharing system may provide a full suite of data integration and analytical capabilities that allow organizations to quickly pivot from fraud identification to incident response and mitigation, all within the same platform. In some embodiments, the healthcare fraud sharing system may use, receive, and/or share healthcare-related data from data sources separate and/or distinct from the entities of the healthcare fraud sharing system. Thus, the healthcare fraud sharing system may allow organizations to proactively detect, investigate, and prevent healthcare fraud.

In some, embodiments, the healthcare fraud sharing system may enable compliance with laws and/or regulations, such as HIPAA, by allowing the separation and/or security of data at two levels. For example, all personally identifiable data may be automatically separated, configured, and/or flagged by access controls at the entity level such that the data may not be shared with other entities. Thus, the sharing of personally identifiable data and/or sensitive information may be impossible.

Sharing of generic schemes through the healthcare fraud sharing system may efficiently combat healthcare fraud threats. In some embodiments, a scheme may be generated by human analysts and/or any participating entity and pushed to the healthcare fraud sharing system for use by other participating entities. In some embodiments, a scheme may be generated by the healthcare fraud sharing system following an attack and/or instance of fraud against any entity using the system. Fraud attacks and/or instances of fraud are described in further detail below. A scheme may differ from a specific fraud attack and/or fraud instance by comprising more abstract characteristics of a fraud attack that may be used to proactively detect other fraud attacks, which is described in further detail below. The schemes may be configured to be enabled and/or implemented on other entities and/or computing systems to defend against and/or combat healthcare fraud attacks from being perpetuated.

### System Overview

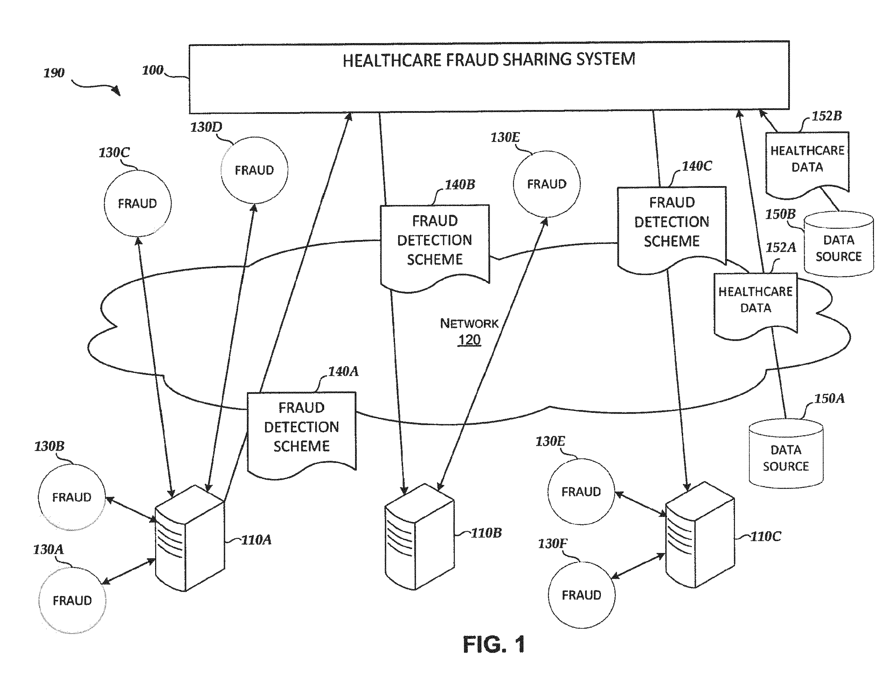

Figure 1 illustrates a healthcare fraud sharing system, according to some embodiments of the present disclosure. In the example embodiment of Figure 1, the healthcare fraud sharing environment 190 comprises a network 120, a healthcare fraud sharing system 100, one or more healthcare fraud attacks 130 (including healthcare fraud attacks 130A, 130B, 130C, 130D, 130E, and 130F in the example of Figure 1), one or more entities 110 (including entities 110A, 110B, and 110C in the example of Figure 1), and fraud detection schemes 140 (including fraud detection schemes 140A, 140B, and 140C). The network 120 may comprise, but is not limited to, one or more local area networks, secure networks, wide area network, wireless local area network, wireless wide area network, the Internet, or any combination thereof. As shown by Figure 1, the healthcare fraud sharing system 100 may share healthcare fraud data and/or schemes with one or more entities 110.

In some embodiments, such as the example embodiment of Figure 1, the healthcare fraud sharing environment 190 further comprises one or more healthcare-related data sources 150 (including data sources 150A and 150B) and one or more healthcare-related data 152 (including healthcare-related data 152A and 152B).

In some embodiments, the healthcare fraud sharing environment 190 may not comprise a network. Some entities, such as, but not limited to, government entities, may not be connected to a network and/or may not want to share healthcare fraud information via a network. Thus, sharing of healthcare fraud information by the healthcare fraud sharing system may occur via physical transport, such as, but not limited to, Universal Serial Bus ("USB") drives, external hard drives, and/or any other type of media storage.

The entities 110 may comprise one or more computing devices. For example, an entity 110 may be institution, such as, but not limited to, a healthcare provider, pharmacy, insurance company and/or organization, a government organization, and/or any organization that can use healthcare fraud related data.

There may be many different variations and/or types of a healthcare fraud attack and/or instance 130. Several examples of healthcare fraud attacks and/or instances are discussed herein, but these are not limiting in the types of other healthcare fraud attacks the healthcare fraud sharing system may be able to detect. For example, a healthcare fraud attack may comprise fraud strategies, such as, but not limited to, upcoding, unbundling, unnecessary procedures/tests, and/or other attack strategies disclosed herein. Healthcare fraud attacks may take place at physical locations, such as healthcare fraud attack 130B, which may illustrate a fraud attack at entity 110A (e.g., fraud at a pharmacy location within an insurance network or a fraudulent claim submitted for services not received, that charge more than they should, or a charge for services for deceased patients, etc.). Healthcare fraud attacks may be received over the network. For example, healthcare fraud attack 130D may illustrate an instance of fraud that is received via the network 120 (e.g., a perpetrator fraudulently purchases and/or orders drugs via the network 120 or a fraudulent claim submission and/or reimbursement process via the network 120). Healthcare fraud attacks and/or healthcare fraud patterns may evolve and/or change over time. Therefore, the healthcare fraud sharing system may be configured to handle different types of healthcare fraud attacks and/or the changes in healthcare fraud attacks over time.

The following are non-limiting examples of healthcare fraud attacks. In an upcoding example, instead of using the appropriate procedure code, a healthcare office may use a procedure code that is more highly reimbursing and/or of higher value. In an unbundling example, instead of using the appropriate procedure code, a healthcare office might break out the component parts of a procedure into individual component procedure codes to obfuscate a non-covered service and/or increase compensation by charging more for the individual procedures than the actual bundled procedure costs. In an unnecessary procedures and/or tests example, patients receive and/or extra tests and/or procedures are reported at an office to increase compensation for that office. In some examples, unnecessary procedures and/or tests may represent healthcare waste, which may be detected by the healthcare fraud sharing system.

In the example embodiment of Figure 1, entity 110A may be attacked by and/or defrauded by one or more healthcare fraud attacks 130A, 113B, 130C, and/or 130D. Entity 110A and/or human analysts of entity 110A may generate fraud detection scheme 140A to be shared with other entities through the healthcare fraud sharing system 100. The same fraud detection scheme 140 may be received by other entities through the healthcare fraud sharing system 100 via the network 120.

In some embodiments, schemes and/or fraud data may be enhanced and/or improved through the use of healthcare-related data 152 from non-entity data sources 150. For example, a fraud detection scheme may detect fraud where a membership identifier is being fraudulently used at a pharmacy and/or healthcare provider for a person on a deceased list. The healthcare fraud sharing system 100 may receive healthcare-related fraud data 152A from a data source 150A via the network 120. In the deceased person example, the healthcare fraud sharing system 100 may receive data from a deceased person master list, which may correspond to the data source 150. Thus, a fraud detection scheme may rely on and/or use data from a deceased person master list to detect a membership identifier associated with a deceased person.

In some embodiments, the healthcare fraud sharing system 100 may receive healthcare-related data 152B from data source 150B that is not connected to the network 120. For example, receipt of the healthcare-related data 152B may occur via physical transport, such as, but not limited to, USB drives, external hard drives, and/or any other type of media storage.

As used herein and as discussed in further detail below, a "data source" may refer to an entity 110, a source of information that is proprietary and/or local to an entity, and/or a non-entity source of information, such as, but not limited to, a public website, public vendor, and/or private vendor of information. Also, as used herein, "healthcare-related data" may refer to healthcare fraud attack data and/or other data from entities and/or data that originates from non-entity data sources. In some embodiments, weighting data may be associated with the data sources, such that the weightings are indicative of a reliability of healthcare related data from the respective data sources.

The healthcare fraud sharing system 100 may operate as a single instance, client server system, or as a distributed system. For example, there may be multiple instances of the healthcare fraud sharing system 100 running simultaneously that communicate through the network 120. In some embodiments, each healthcare fraud sharing system instance operates independently and/or autonomously. In some embodiments, there is a central server of the healthcare fraud sharing system 100 and individual clients of the healthcare fraud sharing system communicate with the central server via the network 120. In the central server example, the central server may contain the open source data, which is described in further detail below. Each participant of the healthcare fraud sharing system 100 may communicate with other participants and/or the central server, such as via a federated search engine (e.g.,

### DINO)

### Schemes

A scheme may comprise code instructions and/or sets of data that enable a healthcare fraud system and/or entity to implement detection and/or defense strategies against healthcare fraud attacks. A scheme differs from specific fraud data in that a scheme may include more abstract characteristics of an attack that can be used to detect other attacks. For example, a scheme may comprise code instructions and/or data representing upcoding behavior based on a pattern of outlier procedures and/or an increase in certain types of complex procedures over time. In different fraud attacks, adversaries may upcode different procedures. Thus, a scheme that identifies upcoding behavior does not necessarily indicate particular procedure codes and/or particular entities that have been implicated and/or attacked by the upcoding behavior, but may include characteristics of the upcoding behavior, such as may be reported by multiple entities, which can be used to detect similar attacks. For example, an upcoding scheme may represent a percentage based threshold for detecting outlier procedures, an increase over time threshold for complex procedures, and/or any other information that may be used by an entity to identify similar upcoding activities. Sharing of an upcoding scheme, whether based on upcoding data from a single entity and/or upcoding data from multiple entities (e.g., as may be developed by the healthcare fraud sharing system 100 in response to receiving upcoding activity data from multiple entities), may enable early detection by the entity and/or the healthcare fraud sharing system of upcoding behavior.

A scheme may be in various formats, such as source code and/or code instructions, a database format, stored procedures, queries, files, XML, JSON, a file format that is proprietary to the healthcare fraud sharing system 100, or any other format, and may be encrypted or have healthcare fraud of any available type, and/or some combination thereof. In some embodiments, a scheme may be enabled on different entities and/or computer systems without modification. For example, two different entities may use different healthcare processing systems (e.g., systems that process healthcare and/or insurance claims). The scheme may be sufficiently abstracted such that, with some configuration data and/or configuration of the entity's computing devices, the same scheme checking for upcoding (or other) behavior may be run on different entities that have different healthcare processing systems. For example, different entities may be configured with the healthcare fraud sharing system to both receive fraud detection alerts based on the same scheme, even though the different entities have different back end systems because they have been configured and/or integrated with the healthcare fraud sharing system.

In some embodiments, schemes and/or the output of schemes, such as healthcare fraud detection data, may be clustered by the systems, methods, and/or techniques disclosed in the Cluster references. For example, related schemes and/or the output of schemes may be clustered as illustrated by U.S. Patent Application Serial No. 13/968,265. A human technician may then view and analyze the cluster of related schemes and/or output of the schemes.

### Example Scheme Sharing

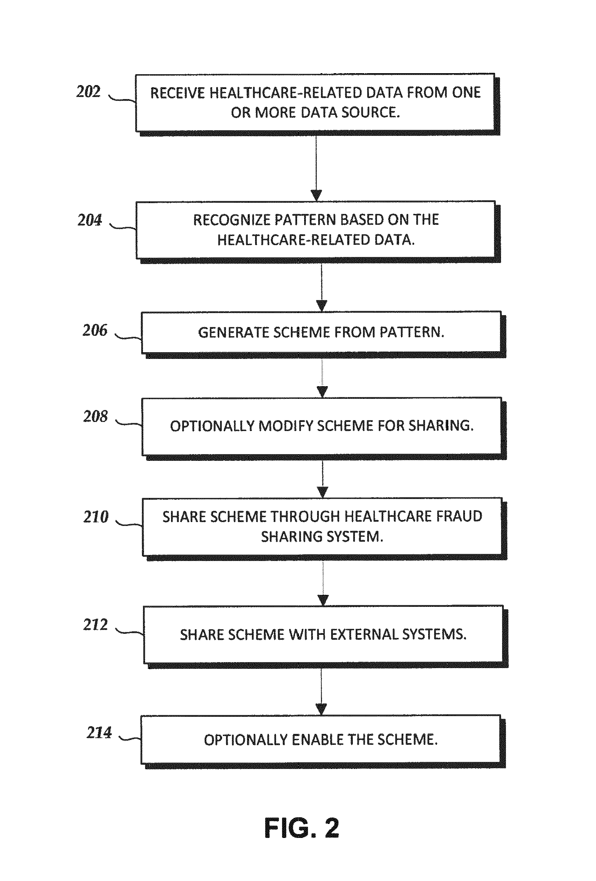

Figure 2 is a flowchart illustrating a scheme sharing process, according to some embodiments of the present disclosure. The method of Figure 2 may be performed by the healthcare fraud sharing system and/or the one or more entities discussed about with reference to Figure 1. Depending on the embodiment, the method of Figure 2 may include fewer or additional blocks and/or the blocks may be performed in order different than is illustrated.

Beginning at block 202, healthcare-related data, such as fraud attack data and/or other data, is received from one or more data sources and/or entities. For example, the healthcare fraud attack may correspond to an upcoding attack and/or instance of fraud that includes hundreds or thousands of office visits and/or procedures that have been identified by a particular entity. The data corresponding to the upcoding attacks may be received by the healthcare fraud sharing system.

At block 204, a pattern may be recognized based on the healthcare-related data. A detected and/or recognized pattern may indicate generalized properties and/or characteristics regarding healthcare fraud attacks. In the upcoding example, a detected pattern may indicate particular procedures for upcoding, percentage thresholds for upcoding, and/or thresholds for particular types of procedures (e.g., complex and/or expensive procedures) over time.

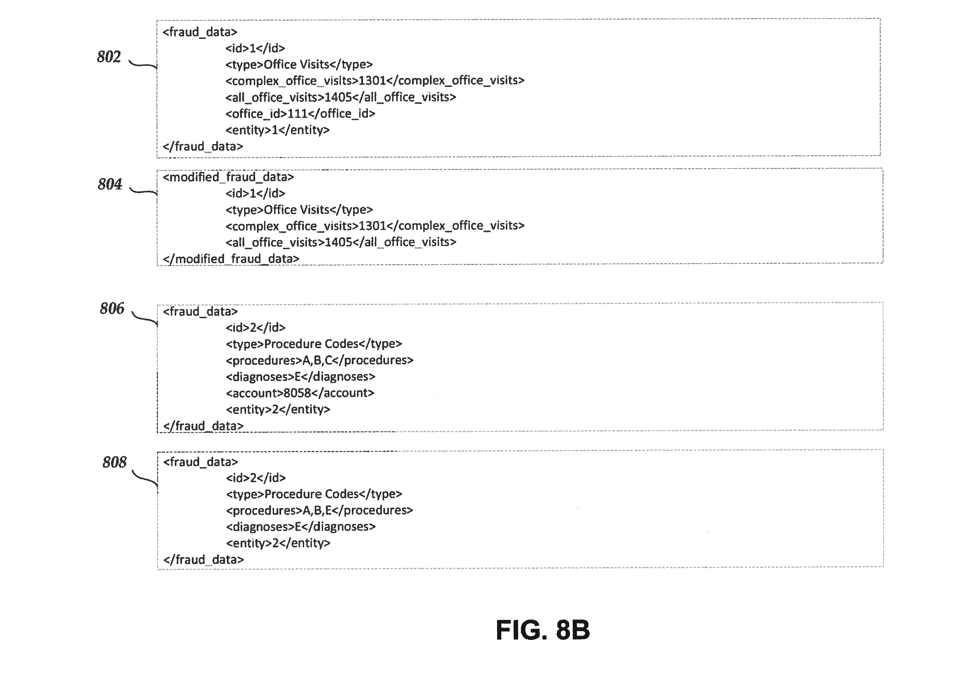

In some embodiments, there may be some variations of recognition of the pattern at block 204. Recognition of a pattern from the healthcare fraud attack at block 204 may be manual or automatic. For example, automatic pattern recognition by the healthcare fraud sharing system may occur from identifying upcoding of particular types of procedures and/or at particular outlier percentage thresholds. The use of outlier percentages is discussed further below with reference to Figure 8B. In some embodiments, human analysts may review the healthcare fraud attacks to recognize a pattern from them.

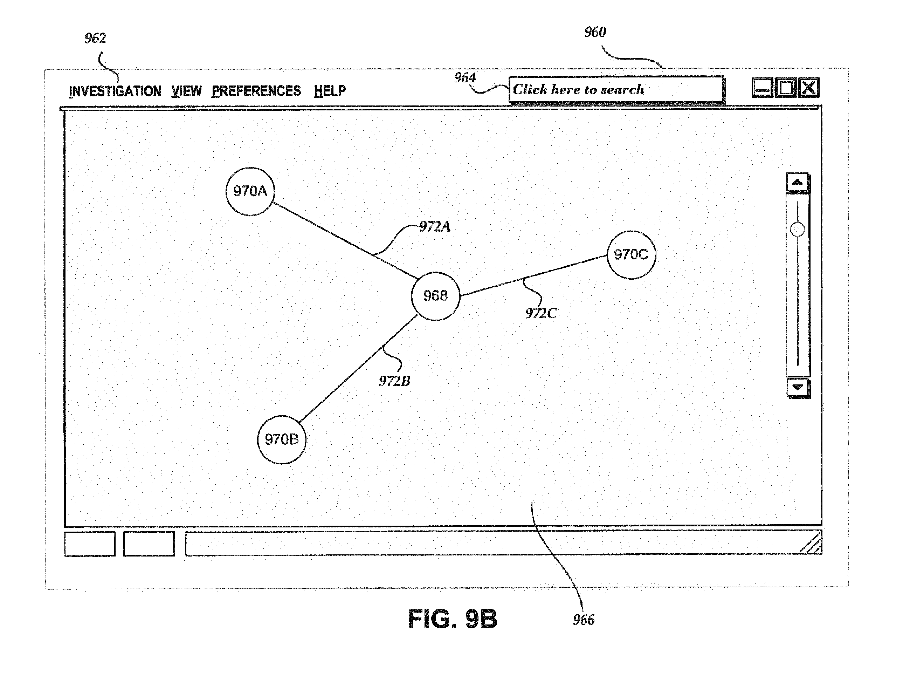

In some embodiments, recognition of patterns occurs by the systems, methods, and/or techniques disclosed in the Cluster references. For example, healthcare attack data may be visualized by user interface clusters, as discussed below with reference to Figure 9B.

At block 206, a scheme may be generated from the recognized pattern. In some embodiments, generation of schemes is automatic, manual, or some combination thereof.

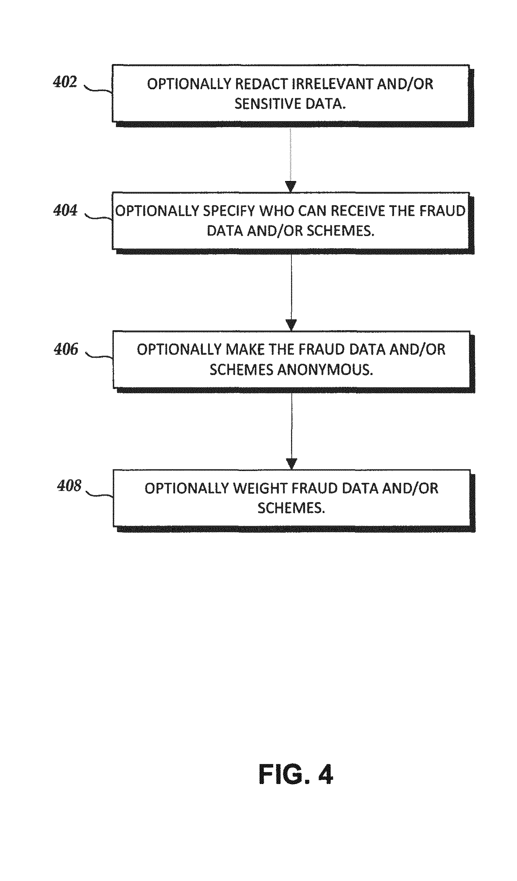

At block 208, the scheme may be optionally modified for sharing, such as in the manner discussed with reference to Figure 4, below.

At block 210, the healthcare fraud sharing system may share the scheme with one or more other entities or external systems. The fraud scheme may be provided by the entity 110 to the healthcare fraud sharing system 100, such as via the network 120 of Figure 1. Depending on the embodiment, the fraud scheme may be shared in various manners, such as via a shared network location that stores the scheme, a direct communication via an email or HTTP communication, or in any other manner.

In some embodiments, sharing of the fraud scheme at block 206 occurs by the systems, methods, and/or techniques disclosed in the Sharing references. For example, a fraud scheme may be shared and/or deconflicted through a replicated database system as illustrated by U.S. Patent No. 8,515,912, thereby preventing duplicate and/or conflicting copies of data. Fraud schemes may also be shared through a database system with multiple ontologies as illustrated by U.S. Patent Application Serial No. 13/076,804. The sharing of fraud scheme may also occur via incremental database replication as illustrated by U.S. Patent Application Serial No. 13/922,437.

In some embodiments, secure sharing through audited activity logs occurs by the systems, methods, and/or techniques disclosed in the Audit reference. For example, sharing activity may be stored in cryptographically immutable audit logs that can be quickly analyzed for suspicious user behavior.

In some embodiments, the clusters generated by the systems, methods, and/or techniques disclosed in the Cluster references and/or other healthcare fraud information may be shared by the systems, methods, and/or techniques disclosed in the Sharing references, other mechanisms illustrated in this disclosure, and/or any other manner.

At block 212, the fraud scheme that is received at the healthcare fraud sharing system 100 is wholly or partially shared with one or more entities 110. For example, if the fraud scheme is received from entity 110A, the healthcare fraud sharing system 100 may share the fraud scheme to entities 110B, 110C, and/ or external systems, such as in accordance with sharing preferences of the entities.

At block 214, the scheme may be optionally enabled by the external system and/or entity.

### Example Fraud Sharing Processes

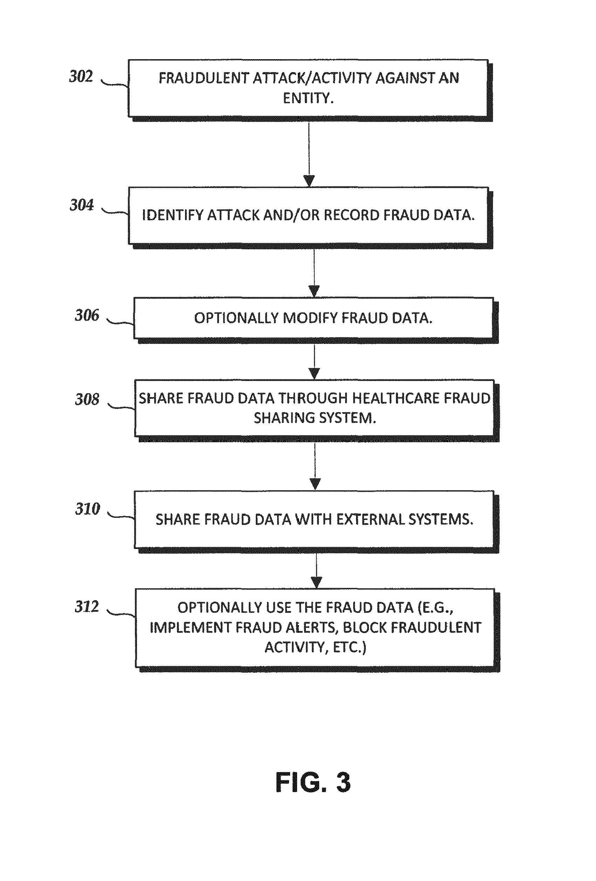

Figure 3 is a flowchart illustrating a fraud attack and/or fraud instance sharing process, according to some embodiments of the present disclosure. The method of Figure 3 may be performed by the healthcare fraud sharing system and/or one or more entities discussed about with reference to Figure 1. Depending on the embodiment, the method of Figure 3 may include fewer or additional blocks and/or the blocks may be performed in order different than is illustrated.

Beginning in block 302, a fraudulent attack and/or activity is made against one of the entities 110. As noted above, various activities may be considered attacks on an entity 110. For example, false and/or unnecessary healthcare procedures may be reported and/or invoiced at the entity.

At block 304, the healthcare fraud attack is identified and/or recorded as fraud data. For example, the entity 110 may identify the use of upcoded, unbundled, and/or unnecessary procedures and/or tests. The fraud data may comprise information about the healthcare fraud attack, such as the procedure codes used, number of procedures, where the procedures took place, and/or identifiers regarding the determined attackers. In some embodiments, the healthcare fraud attack may be identified automatically or the attack may be identified by a human analyst, such as a fraud detection analyst. In some embodiments, attacks are initially detected and/or flagged by one or more systems and/or processes and then a human analyst confirms the detected attack before the fraud data is shared, such as according to the process described below.

In some embodiments, identification of attacks and/or instances of fraud occurs by the systems, methods, and/or techniques disclosed in the Cluster references. For example, related healthcare fraud attacks may be clustered as illustrated by U.S. Patent Application Serial No. 13/968,265. A human analyst may then view and analyze the cluster of related healthcare fraud attacks. Clusters of healthcare fraud attacks may also receive rankings and/or scorings as illustrated by U.S. Patent Application Serial No. 13/968,213.

In some embodiments, fraud data, schemes, and/or other healthcare fraud information may be a data object according to the systems, methods, and/or techniques disclosed in the Ontology reference. For example, fraud data, schemes, and/or other healthcare fraud information may be included in data objects that are included in an ontology, which may be shared with other entities across the healthcare fraud sharing system and/or the data objects remain uniform across the entities they are shared with. In other words, the healthcare fraud sharing system may support a unified data object ontology. Additionally, each entity may support its own data object model and/or ontology that is different from its peer entities. In some embodiments, an ontology may provide a consistent view of fraud data across multiple entities. Another benefit of a unified data object ontology is to prevent duplicate and/or conflicting copies of data objects, and/or to allow for easy de-duplication of data objects.

At block 306, the fraud data may be optionally modified for sharing. For example, information regarding account numbers or personal information, such as social security numbers, may be removed from the fraud data before it is shared with the healthcare fraud sharing system 100. The entity 110 may remove and/or modify data regarding the attack and/or the healthcare fraud sharing system 100 may remove and/or modify data regarding the attack once received from the entity 110 (e.g., as discussed below in block 308).

Next, at block 308, the fraud data may be provided by the entity 110 to the healthcare fraud sharing system 100, such as via the network 120 of Figure 1. The sharing of the fraud data with the healthcare fraud sharing system may be similar to the sharing of the schemes with the healthcare fraud sharing system at block 210 of Figure 2. The fraud data may be in various formats, such as a database format, files, XML, JSON, a file format that is proprietary to the healthcare fraud sharing system 100, or any other format, and maybe encrypted or have healthcare fraud of any available type.

In some embodiments, sharing of fraud data at block 308 occurs by the systems, methods, and/or techniques disclosed in the Sharing references.

In some embodiments, the clusters generated by the systems, methods, and/or techniques disclosed in the Cluster references and/or other healthcare fraud information may be shared by the systems, methods, and/or techniques disclosed in the Sharing references, other mechanisms illustrated in this disclosure, and/or any other manner.

At block 310, the fraud data that is received at the healthcare fraud sharing system 100 is wholly or partially shared with one or more entities 110. The sharing of fraud data with external systems may be similar to the sharing of the schemes with external systems at block 212 of Figure 2.

At block 312, the fraud data may be optionally used by the entities with which the fraud data is shared. For example, the fraud data may be used to proactively detect and/or hopefully prevent similar attacks.

There may be some variations of the optional use of the fraud data at block 312. For example, an entity 110B, may implement healthcare fraud defenses, such as fraud alerts, against the actors and/or behaviors identified in the fraud data. In another example, the entity 110B, may conduct a search for the fraudulent behavior throughout the entity's historical records based on the received fraud data. Thus, the fraud data may be used to identify previous attacks (which may be ongoing) and initiate recovery actions (e.g., flagging accounts, stopping payments, initiating enforcement actions, etc.).

### Modifying Fraud Data And/Or Schemes

Figure 4 is a flowchart illustrating a modification process for fraud data and/or schemes, according to some embodiments of the present disclosure. The method of Figure 4 may be performed in whole, or in part, as part of block 208 of Figure 2 and/or block 306 of Figure 3. Depending on the embodiment, the method of Figure 4 may include fewer or additional blocks and/or the blocks may be performed in order that is different than illustrated.

At block 402, irrelevant and/or sensitive data may be redacted from fraud data and/or schemes. For example, fraud data may initially comprise sensitive personal information, such as, but not limited to, social security numbers, health records, names, birthdates, addresses, etc. An entity may not want to and/or be legally prohibited from sharing such information. An entity may redact and/or remove particular information. Thus, redaction, removal, and/or de-identification may allow an entity to be in compliance with applicable laws and/or regulations. Removal of sensitive information and/or entity specific information, such as internal account numbers, from fraud data, may abstract the fraud data to increase usability by other entities. In some embodiments, redaction of fraud data and/or schemes is automatic, manual, or some combination thereof. For example, there may be a configurable list of fields, such as, name, account number, etc., to be removed from fraud data and/or schemes. Redaction may require approval by a human analyst. In some embodiments, redaction of fraud data and/or schemes may be performed by a human analyst.

At block 404, recipients may be specified for fraud data and/or schemes. For example, an entity may only want to send fraud data to other entities it has close relationships with or entities in a particular vertical market or having other attributes. Therefore, the entity may specify one or more criteria for entities with which fraud data and/or schemes may be shared with through the healthcare fraud sharing system. The sharing data may be provided in any available format, and may apply to sharing of fraud data and/or scheme data from the entity that provides the sharing data. In some embodiments, a human analyst must approve and/or select the recipients of fraud data and/or schemes.

In some embodiments, access controls for replicating fraud data and/or schemes at block 404 occurs by the systems, methods, and/or techniques disclosed in the Sharing references. For example, asynchronous replication of fraud data and/or schemes occur via access control policies illustrated by U.S. Patent No. 8,527,461. Replication of fraud data and/or schemes may occur where databases use different classification schemes for information access control as illustrated by U.S. Patent Application Serial No. 13/657,684.

At block 406, fraud data and/or schemes may be made anonymous. For example, fraud data and/or schemes may comprise the source entity of the fraud data and/or schemes. Thus, an entity may specify whether the sharing fraud data and/or schemes should be anonymous. In some embodiments, there is a global setting and/or configuration for specifying anonymity. There may be a configurable setting enabling anonymity for some recipients but not others. In some embodiments, a human may approve or specify anonymity for each fraud data item and/or scheme that is shared.

At block 408, fraud data and/or schemes may be weighted differently such as based on the entity that provides the fraud data or scheme set (e.g., some entities may be more reliable providing fraud data than others) or based on the type of attack identified in the fraud data or scheme set, and/or other factors. For example, if fraud data indicates a high fraud risk associated with the attack, the healthcare fraud sharing system may assign a high weighting to the fraud data. However, if the reported attack is less malicious and/or from an entity that commonly misreports attacks, a lower weighting may be assigned to the fraud data, such that sharing of the fraud data doesn't introduce false attack alerts in other entities. Thus, in some embodiments, the healthcare fraud sharing system tracks the accuracy, reliability, and/or trustworthiness of reported attacks from respective entities and automatically applies weightings and/or prioritizations to future reports from those entities based on the determined accuracy.

The weightings may be assigned manually and/or automatically. For example, in some embodiments a human analyst specifies whether fraud data and/or schemes are important. These weightings may change over time, as the attacks themselves evolve.

From the receiving perspective of fraud data and/or schemes, an entity may optionally weight fraud data and/or schemes from different entities. Thus, if an entity values fraud data and/or schemes from a different entity highly, the entity may set a high level of priority for anything received from that different entity.

### Sharing Fraud Data and/or Schemes

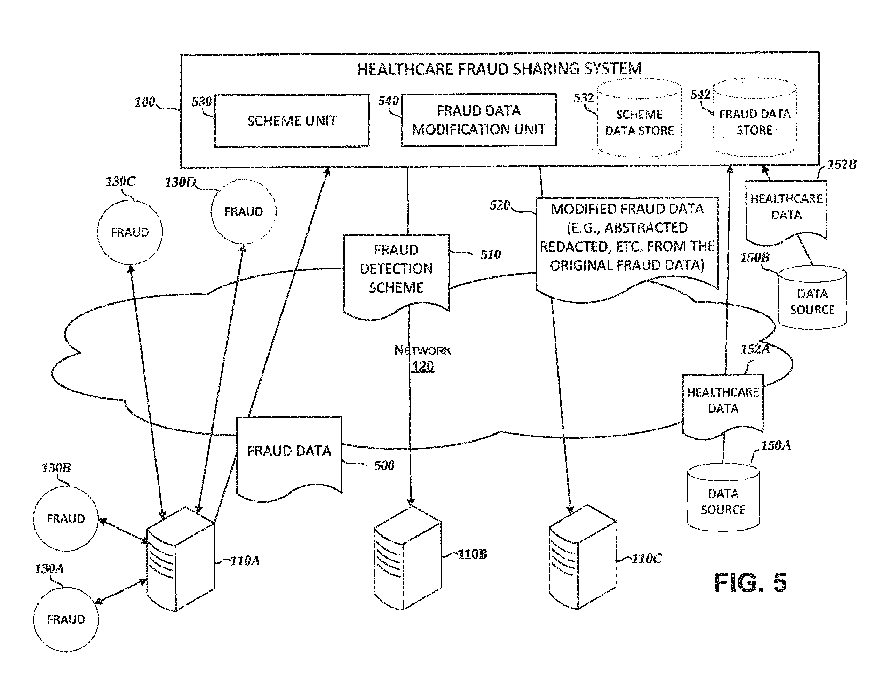

Figure 5 illustrates a healthcare fraud sharing system sharing fraud data, schemes, and/or modified fraud data, or subsets thereof, according to some embodiments of the present disclosure. In accordance with some embodiments of the present disclosure, the healthcare fraud system 100 may comprise a scheme unit 530, a fraud data modification unit 540, a scheme data store 532, and/or a fraud data store 542.

As shown in the example of Figure 5, an entity 110A has been involved in fraud attacks 130A, 130B, 130C, and/or 130D (each comprising one or more transmissions and/or receptions of data from the entity 110A). In this embodiment, the entity 110A, upon identifying the one or more fraud attacks (see, e.g., Figure 2), may send fraud data 500 to the healthcare fraud sharing system 100 through the network 120. In some embodiments, the healthcare fraud sharing system 100 automatically collects fraud data from a healthcare fraud attack.

In this example, the healthcare fraud sharing system 100 generates a scheme and/or modified fraud data based on the fraud data 500 corresponding to the one or more fraud attacks 130, such as by any one or more processes discussed with reference to Figure 3 and/or Figure 4. For example, the multiple attacks 130A, 130B, 130C, and/or 130D illustrated in Figure 5 may be associated with upcoding attacks directed to the entity 110A. The fraud detection scheme 510 may be generated and/or output to other entities by the scheme unit 530. The fraud detection scheme 510 may be stored in the scheme data store 532. The modified fraud data 520 may be generated and/or output to other entities by the fraud data modification unit 540. The modified fraud data 520 may be stored in the fraud data store 542. In the embodiment of Figure 5, the healthcare fraud sharing system 100 shares the fraud detection scheme 510 with another entity 110B through the network 120 and the modified fraud data 520 with another entity 110C. The entities 110B and 110C may change, update, modify, etc., their healthcare fraud measures based on the scheme 510 or modified fraud data 520, respectively.

In some embodiments, the healthcare fraud sharing system 100 may allow for integration with law enforcement agencies (as another entity within the healthcare fraud sharing system 100 or otherwise). For example, an entity may flag a fraudulent actor and share that data with the healthcare sharing system 100, which may automatically notify one or more law enforcement agencies that may initiate a case against that actor. The healthcare fraud sharing system 100 may then allow for the tracking of the law enforcement case by multiple entities throughout the system. Furthermore, other entities may contribute healthcare-related fraud data regarding that fraudulent actor and/or case, which may also be received by the one or more law enforcement agencies.

In some embodiments, the healthcare fraud sharing system 100 may be able to automatically generate schemes based on one or more healthcare fraud attacks. Similar to automatic recognition of healthcare fraud attack patterns, a scheme may be automatically generated from patterns of healthcare fraud attacks, e.g., upcoding and/or unbundling attacks. Schemes may be automatically output by the scheme unit 530. For example, the scheme unit 530 may take as input data regarding healthcare fraud attacks and automatically generate a scheme from patterns recognized in the data.

In some embodiments, a human analyst and/or a team of analysts may review the healthcare fraud attack patterns to generate a scheme. The healthcare fraud sharing system may provide user interface tools to humans for analyzing healthcare fraud attacks and/or creating schemes. For example, schemes may be generated by a human analyst of the user interface of the scheme unit 530. A user interface of the scheme unit 530 may comprise a document processing interface to generate a scheme and/or a cluster user interface, as disclosed herein, to recognize patterns of healthcare fraud attacks.

In some embodiments, a team of analysts may consume all of the healthcare fraud information and/or data from all of the entities of the healthcare fraud sharing system. The analysts may conceive and/or generate schemes to share them with entities through the healthcare fraud sharing system.

In some embodiments, schemes may be generated by entities and shared through the healthcare fraud sharing system. For example, the scheme unit 530 may receive schemes from entities for distribution to other entities through the healthcare fraud sharing system.

The shared fraud data and/or scheme may be modified by the entity 110A and/or the healthcare fraud sharing system 100, such as by any one or more processes discussed with reference to Figure 4. Modification by the attack modification unit 540 and/or storage in the attack storage unit may achieve some of the goals and/or advantages illustrated in Figure 4.

### Data Sources

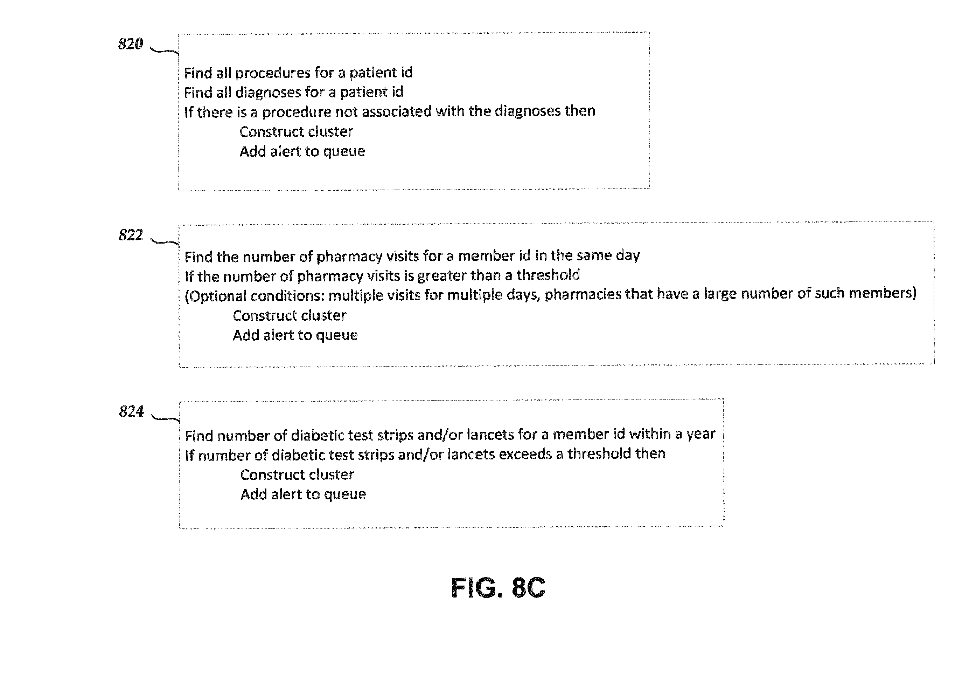

Data from data sources and/or data pipelines may enhance, be used, and/or be included in healthcare fraud detection schemes and/or fraud data that is shared through the healthcare fraud sharing system 100, as is discussed within this application and further below with reference to Figure 8C. In some embodiments, as discussed above, data sources for the healthcare fraud sharing system 100 may refer to entities, sources of information that feed into the entities, non-entity sources of information, and/or hybrid sources of information that combine information from both entities and non-entities (e.g., pharmacy, provider, and/or membership data). Data sources of the healthcare fraud sharing system 100 may be internal to the entity, such as medical claims systems, pharmacy claims systems, authentication call lists, and/or customer relationship management ("CRM") systems.

Non-entity data sources 150 of Figure 1 and/or Figure 5 may provide healthcare-related data 152 to the healthcare fraud sharing system 100. Non-entity data sources may include public websites, information, and/or other open sources of healthcare data. Some non-limiting examples of open source healthcare data may include provider exclusion lists (e.g., OIG exclusion lists), provider licenses, National Provider Identifier ("NPI")/National Plan and Provider Enumeration System ("NPPES"), background reports and/or checks (e.g., private vendor background report), physical mail delivery data (e.g., mail provider drop box locations, mailing store locations), social security death master data, durable medical equipment ("DME") supplier data, law enforcement data sources, United States census data, news data (e.g., news reports on healthcare fraud), public health records, supplier lists, provider quality data, and/or relevant geospatial data. For example, physical mail drop box data 152A may be used by fraud detection schemes 510 to identify fraudulent providers. In the drop box example, if a provider address matches a drop box location, then that match may be an indicator of fraudulent activity because most providers would have a physical office address (and in some cases providers may be required by law to have an actual physical address not just a drop box address).

In some embodiments, the table below illustrates non-limiting examples of data sources that may be used and/or accessed by the healthcare fraud sharing system (and/or an entity) to detect healthcare fraud.

| **Data Source** | **Description** |
|---|---|
| Social Security Death Master File | List of deceased persons managed by Social Security. |
| NPI/NPPES | National provider identifier system provides a unique provider identification number and contact information. |
| Drug Enforcement Administration ("DEA") Active Controlled Substances Act ("CSA") Registrants Database | Registration under the act enables physicians, related practitioners, other established health organizations, pharmaceutical companies, and others to prescribe and/or handle controlled substances. |
| Office of Inspector General ("OIG") Exclusion List | OIG's List Of Excluded Individuals/Entities ("LEIE") provides information regarding individuals and entities currently excluded from participation in Medicare, Medicaid, and other healthcare programs. |
| Centers for Medicare & Medicaid Services ("CMS") Supplier Directory (DME Suppliers) | Provides the names, addresses, and contact information for suppliers that provide services and/or products under healthcare programs. |
| National Drug Code Directory ("NDC") | The Drug Listing Act of 1972 requires registered drug establishment provide the food and drug administration ("FDA") with a current list of all drugs manufactured, prepared, propagated, compounded, and/or processed by it for commercial distribution. |
| AHFS Drug Information Database | The American Society of Health-System Pharmacists ("ASHP") Formulary Service includes drug information on indications, dosage and administration, contraindications, side effects, drug interactions, pharmacology and pharmacokinetics, chemistry and stability, and other information. |
| News Sources (e.g., Fraud Tips) | Extracted entities and/or that appear in healthcare fraud cases reported in the media or by Law enforcement. |
| Excluded Parties List System ("EPLS") | EPLS is an electronic, web-based system that identifies those parties excluded from receiving Federal contracts, certain subcontracts, and certain types of Federal financial and non-financial assistance and benefits. |
| Clinical Laboratory Improvement Amendments ("CLIA") Abuse Reports | List of CLIA entities who have been convicted, had their license suspended, and/or have other sanctions. |
| State Licensing | Each state licensing board and/or department of health may publish a "check your doctor" service to check the status of any given doctor's license and disciplinary actions. |
| Mailing Facility: Mail drop box locations, geocoded | List of drop box locations along with their geographic coordinates. Drop boxes are locations for dropping off packages to be delivered. |
| Private Vendor Fraud Tip Lines | Source for healthcare related fraud tips. |
| FDA Data | Drug related data may be sourced from the FDA Online Label |
| | Repository: Human Prescription Labels, FDA Online Label |
| | Repository: Human OTC Labels, FDA Online Label |
| | Repository: Homeopathic Labels, and/or FDA Online Label |
| | Repository: Remainder Labels (Bulk Ingredients, Vaccines, and some Medical Devices). |

In some embodiments, similar to the weighting of entities, fraud data, and/or fraud detection schemes, data sources may also be weighted. For example, fraud data from a law enforcement agency, such as the Federal Bureau of investigation, may receive a high weighting because data from such a data source is highly trustworthy.

In some embodiments, data from data sources (including non-entity data sources) may be mapped to and/or converted to data objects according to the systems, methods, and/or techniques disclosed in the Ontology reference.

In some embodiments, data may be automatically retrieved from open source data sources (and converted to data objects according to the Ontology reference). For example, where data, such as, but not limited to, excel and/or spreadsheet documents, text files, databases, delimited text files, and/or in any other data formats, is available via a public website and/or interface, the data may be automatically retrieved and imported into the healthcare fraud sharing system. Data from public websites may also be automatically scraped and/or retrieved via text and/or webpage parsing. For example, healthcare fraud news and/or a fraud tip from an online provider may be automatically retrieved, converted to shareable fraud data, and/or used in a fraud detection scheme.

In some embodiments, the healthcare fraud sharing system 100 may be configurable to add new data sources and/or data pipelines. For example, the use of the systems, methods, and/or techniques disclosed in the Ontology reference may facilitate integration of new data sources.

### Example Scheme Generation from Multiple Attacks

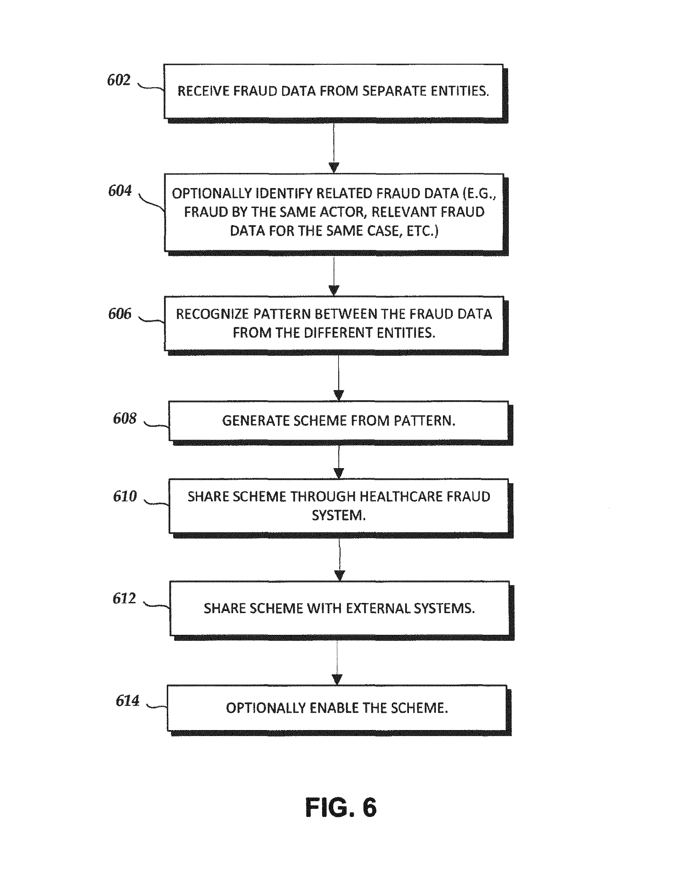

Figure 6 is a flowchart illustrating a scheme sharing process for multiple attacks, according to some embodiments of the present disclosure. The method of Figure 6 may be performed by the healthcare fraud sharing system and/or one or more entities discussed about with reference to Figure 1. Depending on the embodiment, the method of Figure 6 may include fewer or additional blocks and/or the blocks may be performed in order different than is illustrated.

Beginning at block 602, fraud data is received from separate entities. For example, the fraud data from different entities (e.g., entities 130) may correspond to hundreds of upcoding and/or unbundling attacks that have been identified by different entities.

At block 604, a pattern is associated and/or recognized from the fraud data from different entities. A recognized pattern may indicate a particular source of the attack, e.g., a particular perpetrator of the fraud.

At block 606, a scheme may be generated from the recognized pattern. The scheme may be optionally modified for sharing.

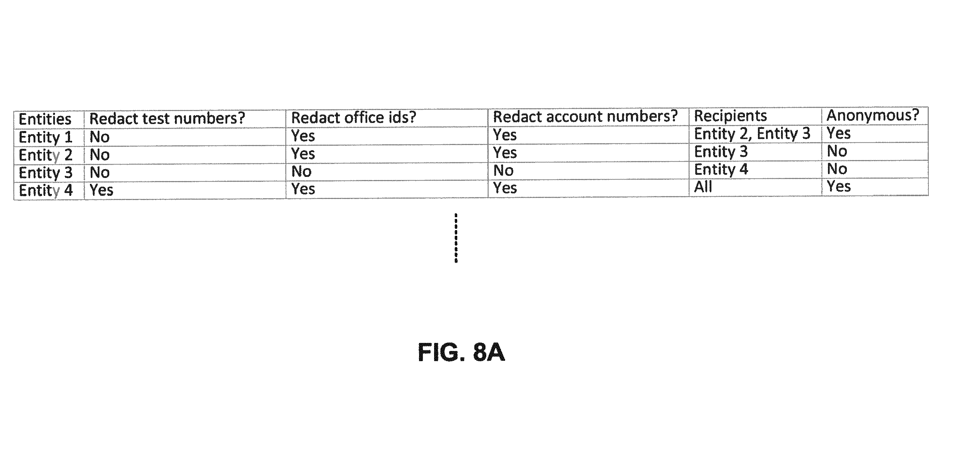

At block 608, the scheme may be shared through the healthcare fraud sharing system. For example, the scheme may be shared with one or more entities that shared fraud data used in generation of the scheme and/or other entities that did not provide fraud data used in generation of the scheme, such as in accordance with sharing schemes (e.g., Figure 8A).

At block 610, the scheme is shared through the healthcare fraud sharing system to external systems. The sharing of the scheme with external systems at block 610 may be similar to the sharing of the fraud data at block 212 of Figure 2.

At block 612, the scheme may be optionally enabled by the external system and/or entity.

### Sharing Fraud Data and/or Schemes from Different Entities

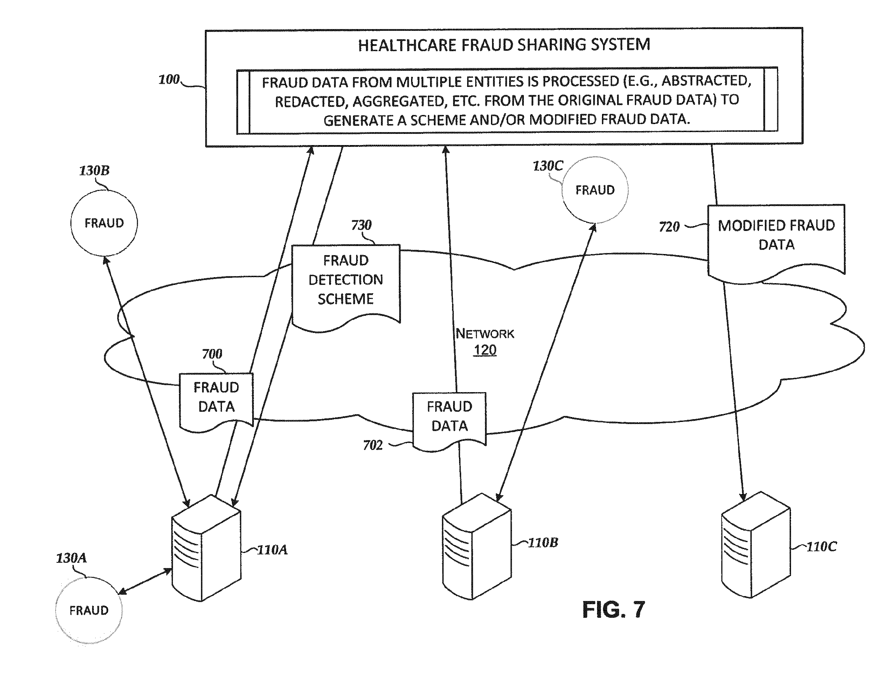

Figure 7 is a block diagram illustrating a healthcare fraud sharing system sharing fraud data, schemes, and/or modified fraud data that has been received from and/or determined based on information from different entities, according to some embodiments of the present disclosure. As shown in the example of Figure 7, the entity 110A has received one or more fraud attacks 130A and/or 130B and the entity 110B has received one fraud attack 130C (although a fraud attack, as used herein, may include one or any number of fraudulent activity and/or instance from and/or to an entity).

In this embodiment, the entity 110B, upon identifying the one or more fraud attacks (see, e.g., Figure 3), may send fraud data 702 to the healthcare fraud sharing system 100 through the network 120. Similar to entity 110B, entity 110A may send fraud data 700, including information regarding attacks 130A and/or 130B to the healthcare fraud sharing system 100. In this example, the healthcare fraud sharing system 100 generates a scheme 730 based on the fraud data 700 from entity 110A and the fraud data 702 from entity 110B. For example, the multiple attacks illustrated in Figure 7 may be associated with upcoding with a similar pattern of attack, such as upcoding within a certain percentage, and/or a set of unnecessary procedures and/or tests.

Scheme generation and/or sharing in Figure 7 may be similar to Figure 5.

The healthcare fraud sharing system 100 may process the fraud data from different entities to share attack schemes, fraud data, and/or modified fraud data. In Figure 7, the healthcare fraud sharing system 100 shares modified fraud data 720 with entity 110C, which may not have been attacked yet by the particular attack(s) 130A-130C that were reported by entities 110A, 110B. For example, the modified fraud data 720 may include upcoding percentage ranges and/or a set of unnecessary procedures and/or tests. The modified fraud data 720 may differ from the fraud data 700 by not having data regarding the particular accounts that were attacked and/or any personal information regarding patients.

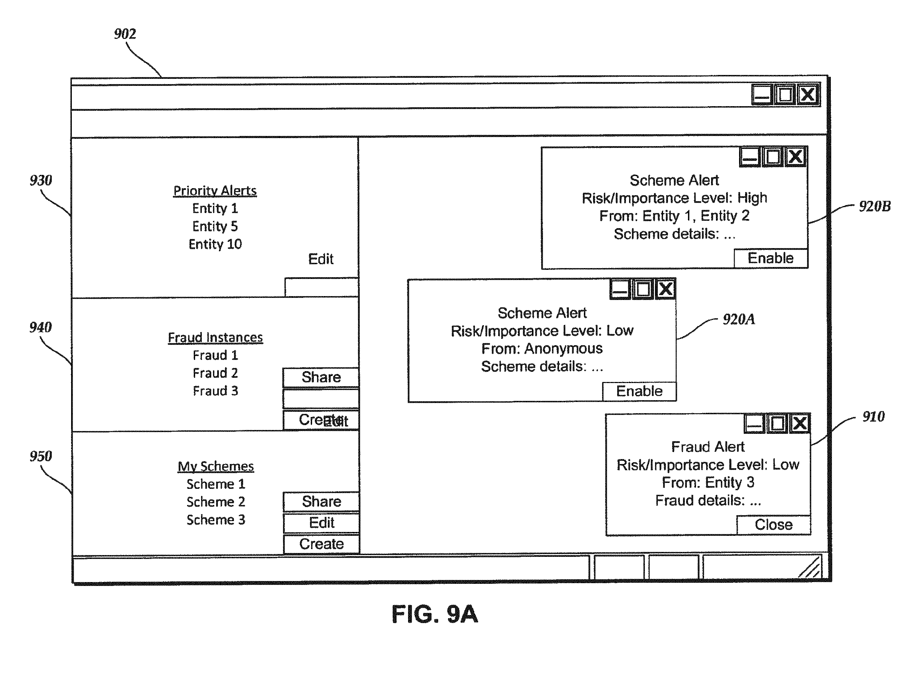

In some embodiments, fraud data may be a "lead" to identify healthcare fraud, which may originate from within an entity and/or be received from another entity such as modified fraud data 720. As described below with references to Figure 9, a particular type of fraud data, one or more fraud objects and/or cluster of fraud objects, and/or lead may be associated with an alert to notify an analyst. A fraud alert may correspond to one or more suspicious fraud data items and/or objects that have been identified by the healthcare fraud sharing system 100. For example, a provider (or customer, and/or person) associated with an entity 110 and/or within entity's 110 network, may be flagged (with a flag object) as suspicious because that provider is prescribing large amounts of a highly addictive drug that is associated with a fraudulent activity, such as oxycodone. Thus, a lead object, flag object, and/or fraud data may be received by one or more entities from the fraud sharing system 100 when suspicious behavior for a provider is identified. In some embodiments, one or more flag objects may be associated with a provider.

### Sharing Tables

Figure 8A illustrates an example healthcare fraud sharing rules and/or redaction table, according to some embodiments of the present disclosure. For example, the healthcare fraud sharing table may be one or more tables in a relational database of the healthcare fraud sharing system. In other examples, the healthcare fraud sharing table may be in various formats, such as a data object format, XML, JSON, a file format that is proprietary to the healthcare fraud sharing system, or any other format. The columns and/or fields shown in the table are illustrative. In some embodiments, there may be additional or less columns and/or fields. The healthcare fraud sharing table may be used to redact and/or modify any property of fraud data, schemes, and/or other healthcare fraud information of the healthcare fraud sharing system. The redaction and/or modification of any property may be possible because fraud data, schemes, and/or other healthcare fraud information may be in a data object format.

As shown in the example of Figure 8A, the healthcare fraud sharing table may be used by the healthcare fraud sharing system (and/or by individual entities in some embodiments) to redact and/or modify fraud data and/or schemes. For example, there are four example entities shown (see the Entities column). Fraud data from a healthcare fraud attack may include the number of tests and/or procedures that were performed at an entity and/or healthcare provide. Thus, the redact test numbers column may be used to remove the actual numbers of test results that were performed. However, in some embodiments, percentages of the procedures and/or types of procedures may be shared instead. In the example, the numbers of test procedures will be removed from entity 4's fraud data and/or schemes. For the entities 1, 2, and 3, the numbers of test procedures may be shared.

As shown in the example table of Figure 8A, there may be other columns for redacting or removing other data from fraud data and/or schemes. For example, specific office and/or pharmacy identifiers, account numbers, and/or personal information may be removed and/or redacted. As discussed above, a healthcare fraud sharing and/or redaction table or equivalent method or device may be useful to redact personal information as required by law, such as the requirements of HIPAA.

The healthcare fraud sharing table may also be used to specify recipients for fraud data and/or schemes. For example, as shown in Figure 8A, entity 1 has recipients: entity 2 and entity 3. Thus, the default recipients of entity 1 are entities 2 and 3 for sharing fraud data and/or schemes. As shown in the example table, entity 4 may share all of its fraud data and/or schemes with every entity in the healthcare fraud sharing system.

As shown in the example table of Figure 8A, there may be a setting to make an entity anonymous while sharing fraud data and/or schemes. Entities 1 and 4 may share fraud data and/or schemes with other entities anonymously. For example, with the anonymous setting enabled, entity 1 can share fraud data and/or schemes with the healthcare fraud sharing system, which may then share that fraud data, schemes, and/or other scheme generated based on the data received from entity 1, with other entities without identifying entity 1 as a source of the fraud data.

### Example Fraud Data, Modified Fraud Data, And/Or Schemes

As previously illustrated, fraud data, modified fraud data, and/or schemes may be in various formats and/or combinations of formats and are not limited to the example formats shown in Figures 8B, 8C, and 8D.

Figure 8B illustrates example fraud data and/or modified fraud data based on healthcare fraud attacks, according to some embodiments of the present disclosure. For example, healthcare fraud attacks such as upcoding may be identified by looking at particular outlying percentages. As used herein, "outlying" or "outliers" may mean a percentage, number, and/or instance that is different from the standard, norm, median, and/or average. The entity's computing device and/or the healthcare fraud sharing system may aggregate data from multiple entities and/or sources to gather sufficient data to identify outliers. An outlying percentage may include the number of complex office visits and/or procedures divided by the total number of office visits and/or procedures as compared to other office numbers and/or percentages. In some embodiments, if complex procedures at an office are a majority of that office's procedures, such as greater than ninety percent, and/or are an outlier compared to other similar offices (e.g., ten percent at one office compared to one or two percent at other offices) then that percentage may indicate healthcare fraud. The example healthcare and/or fraud data 802 includes a data identifier (in the <id> element), and identifies the type of data (in the <type> element), number of complex office visits (in the <complex_office_visits> element), number of total office visits (in the <all_office_visits> element), an office identifier (in the < office_id> element), and entity providing the fraud data (in the <entity> element>).

The healthcare fraud sharing system may modify the fraud data 802, such as in the manner discussed with reference to Figure 4 and/or Figure 8A, to output modified fraud data 804. As illustrated, modified fraud data 804 may not contain the source entity of the fraud data (e.g., the <entity> element is removed) and/or the office identifier (e.g., the <office_id> element is removed) of the source entity, which may correspond to the preferences illustrated with reference to the sharing table of Figure 8A. For example, the sharing table of Figure 8A specified that entity 1 should remain anonymous and redact office identifiers. In another embodiment, entity 1 may include its identifier (e.g., include the <entity> element) and the healthcare fraud sharing system may anonymize the fraud data by not indicating to other entities with which the fraud data is shared that the fraud data came from entity 1. In this way, data that is received by the healthcare fraud sharing system may still determine reliability of information based on source of information, even without sharing the exact identity of the source with other entities.

In another example, a healthcare fraud attack may be an unnecessary procedure and/or test as identified by the types of procedures administered and diagnoses. For example, an unnecessary procedure fraud attack may be identified where one of procedures A, B, or C (illustrated by the <procedures element>) is based on diagnoses D or E (illustrated by the <diagnoses> element). Similar to the previous example, an entity may send fraud data 806 to the healthcare fraud sharing system and/or the healthcare fraud sharing system may modify the fraud data 806 to output modified fraud data 808. Unlike modified fraud data 804, which may be anonymous, modified fraud data 808 may contain the source entity of the fraud data, which may correspond to the preferences illustrated with reference to the sharing table of Figure 8A. For example, the sharing table of Figure 8A specified that entity 2 should not be anonymous. Similar to modified fraud data 804, modified fraud data 808 does not contain the internal IP addresses of entity 2, which may correspond to the preferences illustrated with reference to the sharing table of Figure 8A.

In some embodiments, schemes may be in a similar format to the healthcare fraud data shown in Figure 8B.

Figure 8C illustrates example schemes 820, 822, and 824 in a format comprising code instructions, according to some embodiments of the present disclosure. In some embodiments, schemes may be complex enough such that their expression may be in a format comprising code instructions. The executable code instructions shown in Figure 8D are illustrative pseudocode and, thus, may not correspond to any specific programming language or be executable in the format shown. Executable code that performs the functions outlined in schemes 820, 822, and 824 may be provided in any available programming language.

Example scheme 820 includes code configured to detect healthcare fraud attacks for unnecessary procedures and/or tests. As discussed above, in an unnecessary procedures and/or tests healthcare fraud attack, there may be procedures performed with diagnoses that are not associated with those specific procedures. For example, cosmetic surgery and/or a cosmetic procedure coupled with a diagnosis of low testosterone. Thus, the scheme 820 includes code instructions that cause an entity's computing device and/or the healthcare fraud sharing system to find all procedures and diagnoses for a particular patient identifier. In some embodiments, the patient identifier may be sufficiently redacted and/or disassociated with a patient by the healthcare sharing system to comply with privacy laws, such as HIPAA. The tests and/or procedures may be iterated through and/or checked to see if there is not an association between the diagnoses and the tests and/or procedures. In some embodiments, a data store may store all of the valid diagnoses and procedures associations. If there is a procedure not associated with a diagnoses then a cluster may be constructed, such as by using a patient identifier and/or procedure/diagnoses combination as seeds (see the Cluster references), and an alert may be added to a queue. Thus, the scheme 820 may enable the detection of unnecessary procedures and/or tests healthcare fraud attacks.

Example scheme 822 includes code to detect healthcare fraud attacks for stolen pharmacy membership identification or identifiers ("IDs"). In a stolen ID fraud attack, membership IDs may be stolen and/or shopped around to generate revenue for pharmacies and/or healthcare providers. An indicator of stolen ID fraud may include a member visiting multiple pharmacies within the same day. Other indicators of ID fraud may include other characteristics of pharmacies, such as pharmacies that are independent, pharmacies that are located very far from each other and/or from the purported member address, and/or pharmacies dispensing common drugs that should be reasonably available at a single location. For example, the code instructions shown in 822 cause an entity's computing device and/or the healthcare fraud sharing system to retrieve the number of pharmacy visits for a membership identifier in the same day (as discussed above, the membership identifier may be redacted and/or disassociated with personal information to comply with applicable privacy laws). If the number of pharmacy visits is greater than a particular threshold than a cluster may be constructed (see the Cluster references) and an alert may be added to a queue. For example, if the visit threshold was set to a number, such as three, then an alert would appear if there was more than visits in a day for a particular membership identifier. In some embodiments, there may be further optional conditions for generating clusters and/or adding different levels of alerts based on additional factors. Such factors may include a membership identifier being associated with multiple days of multiple pharmacy visits and/or pharmacies that have a large number of stolen ID alerts as previously specified. Thus, the scheme 822 may enable the detection stolen ID healthcare fraud attacks.

Example scheme 824 includes code to detect excessive and/or inappropriate usage of drugs and/or procedures. For example, a member may receive (or seem to receive) excessive quantities of drugs and or procedures. This may represent fraud on the part of the member (so they can resell) and/or fraud on behalf of the provider/pharmacy (to increase compensation via ghost billing). Schemes to detect such fraud may be done on the basis of a particular threshold and/or known regulations or limits of drugs and/or procedures. For example, the code instructions shown in 824 cause an entity's computing device and/or the healthcare fraud sharing system to retrieve and/or find the number of diabetic test strips and/or lancets for a membership identifier within a year. If the number of diabetic strips and/or lancets exceeds a threshold then a cluster may be constructed (see the Cluster references) and an alert may be added to a queue. The detection of other excessive and/or inappropriate usage of drugs and/or procedures, such as antiretroviral drugs, may be accomplished in a similar manner to that illustrated by scheme 824.

In some embodiments, schemes may comprise various formats and/or combinations of formats. For example, an upcoding scheme may comprise executable code instructions and/or XML documents. In the upcoding example, the executable code instructions may comprise programming logic to detect upcoding attacks and/or instances of fraud. The XML documents may comprise parameters and/or configurations. In the upcoding example, the XML documents may comprise parameters for checking different upcoding percentages, e.g., a threshold percentage for a notification of fraud. Thus, both the code instructions and the parameterized format, such as, but not limited to, XML, may be shared through the healthcare fraud sharing system.

In some embodiments, the table below illustrates non-limiting examples of schemes that may be used, generated, and/or shared by an entity and/or the healthcare fraud sharing system to detect healthcare fraud.

| **Scheme** | **Description** | **Scheme Logic** | **Examples** |
|---|---|---|---|
| Upcoding | Instead of using the appropriate procedure code, providers use one that is more highly reimbursing. This can be detected by looking at outlying percentages (e.g. % complex office visits/all office visits) or by looking at codes that a provider increasingly uses over time (e.g. a provider slowly increases the number of complex office visits over time) | 1) Percentage based %[A]/[A,B,C] is an outlier | **Office visits** Outlying values for %Office A/Office B, C, D, ... |
| | | 2) Increase over time | |
| | | #A ∥ %[A]/[A,B,C] has seen significant increase over time | |
| Unbundling | Instead of using the appropriate procedure code, providers break out the component parts of a procedure into individual component procedure codes to either obfuscate a non-covered service or increase compensation | One of [A,B,C] and one of [D,E] for the same patient on the same day/week | **Injection Nerve Block** |
| | | | One electrical stimulation and more than 2 injections on the same day for the same patient |
| Unnecessary procedures/tests | Patients receive unnecessary tests and procedures only given by providers in order to increase compensation. | One of [A,B,C] for patients with diagnoses [D,E] | Outlying spending on lab claims for patients with diagnoses for lumbago and long term use meds |
| | | Outlying spending on procedures [A,B,C] for patients with diagnoses [D,E] | |
| Pill Mill Pharmacies | Pharmacies attempt to increase legal and illegal compensation by selling large amounts of expensive drugs or diverting drugs to the street. This behavior can be detected with several flags. Additionally, collusion with prescribing providers as well as drug addict members or stolen ids can occur to create more complex rings. | Highlight independent, retail pharmacies that satisfy the highest number of following flags: | **Grey Market drug diversion** |
| | | | Independent, retail pharmacies |
| | | Outlying percentage of [A]/[A,B] | Outlying percentage of % branded drugs |
| | | | Outlying percentage of denied claims |
| | | Outlying spending on certain drugs | Outlying spending on grey market drug list |
| | | Is located in [zip codes] | Located in a fraud hotspot defined by [zip codes] |
| Frequent Flyers/Stolen ids | Member ids can often be stolen and shopped around to generate revenue for pharmacies or providers. An indicator of this behavior is members visiting multiple pharmacies in the same day. Other indicators of fraud include pharmacies that are independent, located very far from each other and from the purported member address, and/or each dispensing common drugs that should be reasonably available at a single location. | Members that visit >x distinct pharmacies in same day | **Frequent Flyers** |
| | | | Looked at members who visited >5 distinct pharmacies in a day |
| | | Members with multiple days where above criterion is met | |
| | | Pharmacies that have a large number of such members | |
| Excessive/inappropriate usage drugs | Members receive (or seem to receive) excessive quantities of drugs or procedures. | More than x units of [A,B,C] procedures for same member over a time window of 1 day/week/month/year | **Diabetic test strips** |
| | | | Members receiving more than 1200 diabetic test strips and lancets in one year across pharmacy and medical claims |
| | This can represent fraud on the part of member (so they can resell) or provider/pharmac y (to increase compensation visa ghost billing). This is most often done from the basis of a known regulation or threshold. (e.g. max # of diabetic test strips/month as mandated by CMS, appropriate combinations of antiretrovirals for effective therapy) | If any pair of [[A,B], [A,C]] drugs appears together for same member over time window etc. | A4253 = test strips; 1 unit = 50 strips |
| | | | A4259 = lancets; 1 unit = 100 lancets |
| | | If one of [A,B,C] but no D in same day for same member | pharmacy claims |
| | | | Test strips - NDC codes [...] = 50 strips/each |
| | | | Test strips - NDC codes [...] = 50 strips/each |
| | | | **Antiretrovirals** |
| | | | >5 antiretrovirals in day OR |
| | | | Doesn't have Ritonavir when they have another Antiretroviral OR |
| | | | has an individual drug that is redundant since it is contained in a combination drug they are also taking |
| Double Billing | Certain drugs and other items can be billed on both pharmacy and medical claims. Providers bill both to increase compensation knowing that the systems aren't integrated. | One of [A,B,C] (medical) and [C,D] (pharmacy) on the same day (+/buffer) for the same patient | **Trastuzumab/injections** |
| | | | Use of medical procedure code and NDC code on the same day for the same patient, then looking at patients with multiple occurrences of this behavior. |
| | | | **Herpes Zoster Vaccine** |
| | | | Use of medical procedure code and NDC code on the same day for the same patient, then looking at pharmacies and providers that are repeat offenders and thus good candidates for a marquee case. |
| Sanctioned and Invalid entities | Sanctioned providers, closed-down pharmacies, and deceased members can have their ids stolen or continue to operate and bill claims. Some pharmacies simply change their names and open up at the same address. Analysis for this scheme usually involves pulling in an external data source. | For a list [[A, date], [B, date]] find all claims where provider/member = A and date_of_service > date (or for address version) | **Deceased members** |
| | | | Find claims for members that occur after their listed date of death (often using Social Security data) |
| | | | **Sanctioned providers** |
| | | For a list [[A, date], [B,date]] find all claims where provider/member = A.address and date_of_service > date | Find claims for providers on OIG exclusion lists or other sanction lists to highlight known fraudulent providers at the state or government level in the private network |

In some embodiments, additional details for example schemes and/or other example schemes may be provided in the table below. As used herein, a "category" of schemes may refer to a grouping of related schemes. In some embodiments, a scheme may be in more than one category, may be described more generally or specifically, and/or have more than one name.

| **Name** | **Category** | **Description** | **Details/Examples** |
|---|---|---|---|
| Excessive use of antiretrovirals | Excessive/inappr opriate usage | Antiretrovirals are expensive drugs used for treatment of HIV. More than 5 of these drugs in one day is highly unlikely to correspond to appropriate usage. | Inappropriate dosage/use rules: |
| | | | ▪ regimen tablets with overlapping ingredients |
| | | | ▪ combinations not to be used for clinical reasons |
| | | | ▪ certain drugs not used with ritonavir |
| | | There are also additional rules that can be used | |
| | | | Find people with many inappropriate dosage flags that have the maximum amount of prescribed antiretrovirals (especially on the same day) in order to maximize potential returns. |
| Diabetic Test Strips & Lancets | Excessive/inappr opriate usage | CMS or another data source may publish specific guidelines on the maximum amount of diabetic test strips and lancets allowable without special written consent from a physician. Patients who bill both on Part C and D and exceed the yearly threshold for these supplies (1200/year or 100/month) are flagged. | test strips = 50 per NDC code |
| | | | lancets = 100 per NDC code |
| | | | lancets = A4259 (100 per unit) |
| | | | strips = A5253 (50 per unit) |
| | | Practical considerations: Patient gets monthly or even quarterly deliveries of test strips and other supplies, so looking at only people who exceed threshold for one month or other small timeframes can be noisy and/or inaccurate. In some embodiments, one approach may be to aggregate over a whole year or longer (e.g. 1200/year) and include a buffer (maybe only look at over >=1500/year). | |
| | | Additionally, the larger recoveries happen at the level of finding pharmacies or DME supply providers that have a large amount of such patients. This can indicate maybe ghost billing (they steal patient info and just bill) and/or exploiting of patients who do not remember their orders. Another indicator may be to look for the top providers and/or pharmacies that have lots of members that have too many test strips. | |
| Double Billing (General) | Double Billing | Match pharmacy claims (drugs/NDC codes) to medical claims (procedure codes) on a per patient level to detect double billing. | N/A. |
| Injection Nerve Block | Unbundling | In order to mask a non-covered experimental procedure, some providers instead bill for several injections as well as electrical stimulations for the same patient on the same day. | N/A. |
| Deceased Members | Deceased Members | Find claims for a member that fall after their date of death. | N/A. |
| Office Visits Impossible Day | Impossible Visits | Each type of office visit has an approximate time in minutes associated with it (e.g. Office A = 45 minutes). Using this, find providers that have "impossible days" with regard to the aggregate time of office visits they billed. In a globetrotter example, office visits by the same person that would be physically impossible for a single person to make. For example, office visits a hundred miles from each other within minutes. | N/A. |
| Outlying percentage | Upcoding | Find providers that bill an outlying percentage of %Office A/Office B, C, D, etc. (this is likely an instance of upcoding). | N/A. |
| Pharmacy Flags | Pill Mill Pharmacies | Generate flags that indicate suspicious activity for pharmacies (e.g. high percentage branded drugs, outlying high or low denials, etc.), and then surface/alert for pharmacies who have many different flags. | N/A. |
| Frequent Flyers | Stolen ids | Find members visiting several pharmacies in a single day (and then members/pharmacies that have many instances and high total amounts paid for these situations) | N/A. |
| Trastuzumab Excessive usage/double billing | Double Billing | N/A. | Trastuzumab NDC codes: |
| | | | 50242013460 |
| | | | 50242013468 |
| | | | 50242005656 |
| | | | Trastuzumab HCSPS: J9355 |

In some embodiments, other example schemes may be provided in the table below.

| **Name** | **Category** | **Description/Examples** |
|---|---|---|
| Suspicious patient addresses | Stolen ids | Find patient addresses far away from locations of service. |
| Non-qualified prescribers | Excessive/inappropriate usage | Find providers with the wrong specialties prescribing specialized drugs or opiates for long periods of time. |
| No dose titration | Excessive/inappropriate usage | Find members who immediately get high dose opiates, pain, and/or anti-anxiety medications without first starting with lower doses (e.g. scripts for oxycodone 80mg without a history of being on 20mg, 40mg). |
| Exceeding recommended Morphine Equivalent Dosage ("MED") | Excessive/inappropriate usage | Find members getting more than the recommended Morphine Equivalent Dosage per day, particularly for prescriptions not written by hematologists, oncologists, anesthesiologists, neurologists, or other pain specialists. |
| Overlapping drug regimens | Excessive/inappropriate usage | Find members with identical scripts across different providers for certain key drugs (e.g. opiates). |
| | | Examples include: |
| | | Overlap of 2 or more different short-acting opioids from different prescribers for > 90 days. |
| | | Overlap of 2 or more different long-acting opioids from different prescribers for > 90 days. |
| | | |
| Prescription Drugs | Provider and Pharmacy Profiling | Large quantities of controlled substances claims (especially oxycodone, methadone, hydrocodone, hydromorphone, meperidine, morphine, oxymorphone and promethazine with codeine) with no or few medical claims for the patients. Breakdown by physician specialty, especially primary care providers ("PCPs"). Also, look if there are aberrant prescribing practices with medical billing such as up-coding. |
| Prescription Drugs | Provider and Pharmacy Profiling | Emergency room and hospital overdoses, trended by prescribers. |
| Prescription Drugs | Member Profiling | Opioid dependence diagnosis with continued opioid use. |
| Non-qualified Prescribers | Provider and Pharmacy Profiling | Use taxonomy to identify clinicians who should not be prescribing high dose narcotics over extended periods of time (e.g. OB/GYNH, Peds, GPs). |
| Prescription Drugs | Provider and Pharmacy Profiling | Unusually high number of patients prescribed high-cost NON narcotic brand name drugs referred to as "Hot Drugs". These drugs are generally brand name drugs that do not require prior authorization, between a range of cost (e.g., $100 - $1000). |
| Suspicious Narcotic Combination(s) | Provider and Pharmacy Profiling | Large amounts of "trilogy" mix of narcotics, benzos, muscle relaxants for same patients especially if not prescribed by a pain management physician. |
| Prescription Drugs | Provider and Pharmacy Profiling | Very large quantities of low strength opioids when higher strengths are available. For example: quantity of oxycodone 20mg when member could be taking 80mg at lower quantity. This scheme is common for both long acting and short acting opioids. |
| Prescription Drugs | Provider and Pharmacy Profiling | Controlled substance scripts from dentists for > 10 days supply. |
| Prescription Drugs | Provider and Pharmacy Profiling | Long acting narcotics written by dentists. |
| Prescription Drugs | Provider and Pharmacy Profiling | Identify prescribers who have higher than average opioid prescribing patterns (high MED average) for their region, specialty. Compare like specialty to like specialty and like demographics to like demographics |
| Inappropriate Prescription | Provider and Pharmacy Profiling | Oral fentanyl products without a cancer diagnosis. |
| | | Fentanyl patches prescribed in doses reflecting 48 hour dosing instead of 72 hours. This may be related to healthcare waste. |

### Flag Objects/Benchmark Fraud Data

As described above, flag objects, fraud data, and/or fraud detection schemes may be used to identify suspicious actors (e.g., person or providers) that match one or more flags. A flag object may be flexible, such that, flag objects may be shared through the healthcare fraud sharing system 100 and may identify any object matching one or more property values and/or thresholds as indicated by the flag object. In some embodiments, flags and/or flag objects may be associated with criminal history (e.g., convictions), percentile rankings, and/or other properties of data objects. For example, there may be schemes and/or flag objects for detecting one-degree of separation, such as owning shares in a business, from a previously convicted felon. In some embodiments, the flag objects may have various levels of severity and/or be associated with different types of fraud detection schemes.

In some embodiments, the healthcare fraud sharing system 100 shares and/or provides a large amount of healthcare-related data that may be used as benchmarks for fraud identification. Benchmark data may be used to identify outliers, which may be indicative of suspicious and/or fraudulent activity. For example, a provider maybe flagged as suspicious when a provider is within the 90^{th} percentile of all providers providing a particular drug and/or type of drug (e.g. oxycodone). New insights and/or fraudulent activity may be discovered because the healthcare fraud sharing system 100 provides access to a large pool benchmark data than would otherwise be available to an entity. For example, a provider of an entity for a particular category may be only in the 50^{th} percentile for that particular entity, however, when compared nationwide, that provider may be within the 99^{th} percentile and the provider may be flagged within the healthcare fraud sharing system 100. Other non-limiting examples of benchmark data that may be used to identify fraud include average provider hours billed in a day (where high and/or impossible billed hours may indicate fraud), the highest billers of a certain procedure code compared to a peer group, suspicious billing characteristics and/or combinations of billing behaviors that emulate previously known fraudulent providers (such as a pharmacy billing a high percentage of branded medications but a having a low patient co-pay amount), percent of payments to particular types of drugs (e.g. highly addictive drugs), and/or seasonal drug purchasing patterns (where high volume purchases of a drug off-season may indicate fraud).

### Example User Interface

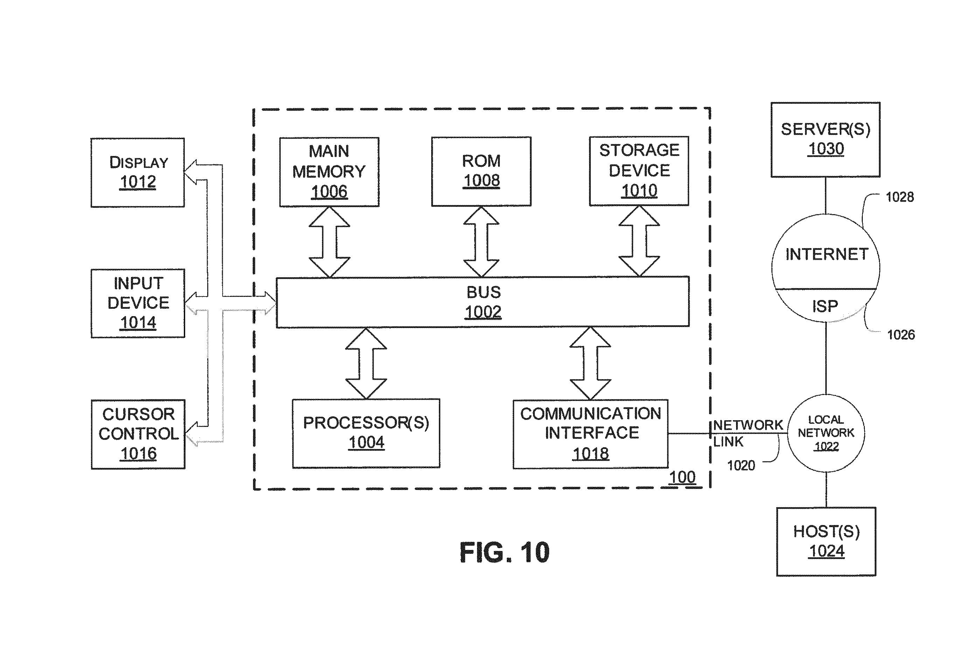

Figures 9A and 9B illustrate example user interfaces of the healthcare fraud sharing system, according to some embodiments of the present disclosure. In some embodiments, the user interfaces described below may be displayed in any suitable computer system and/or application, for example, in a web browser window and/or a standalone software application, among others. Additionally, the functionality and/or user interfaces of the system as shown in Figures 9A and/or 9B may be implemented in one or more computer processors and/or computing devices, as is described with reference to Figure 10.

Referring to Figure 9A, the example user interface 902 comprises healthcare fraud alerts 910, scheme alerts 920A-B, priority alert window 930, fraud attacks window 940, and/or schemes window 950.

In operation, a human analyst may view healthcare fraud attack alerts through the user interface 902. For example, when an entity shares healthcare fraud data with the entity viewing the user interface 902, a healthcare fraud attack alert 910 may appear. The healthcare fraud attack alert 910 may display a risk/importance level, and/or "from" entity indicating the source of the healthcare fraud attack alert. The healthcare fraud attack alert 910 may also display details of the alert and/or a link to the details. For example, healthcare fraud attack alert details may include the pharmacy identifier, member identifier, procedure codes, types of procedures, outlier percentages, and/or the type of attack, e.g., upcoding, unbundling, etc.

In the example of Figure 9A, a human analyst may also view scheme alerts through the user interface. For example, when a scheme is shared in the healthcare fraud sharing system, scheme alerts, 920A and 920B may appear. Scheme alerts 920A and 920B may be similar to healthcare fraud attack alert 910, but instead of healthcare fraud attack details, scheme alerts 920A and 920B may display scheme details. In an impossible days example, scheme details may include the number of visits at a number of pharmacies, the estimated and/or actual time of each visit, and/or the total time of all the visits exceeding a threshold number of hours (e.g., twenty-four hours). The scheme alerts windows 920 may have an "enable" option that activates the scheme as previously described. In some embodiments, scheme alerts 920A-B and/or fraud alerts 910 may be displayed to a user in a list form.

In some embodiments, the healthcare fraud attack alert 910 may have an "enable" option that activates the healthcare fraud attack alert similar to activating a scheme. For example, enabling a healthcare fraud attack alert automatically sets alerts and/or detection of malicious and/or "flagged" providers, pharmacies, and/or member identifiers specified in the healthcare fraud attack alert. The healthcare fraud sharing system may automatically generate a scheme from the healthcare fraud attack alert when the healthcare fraud attack alert is enabled. In some embodiments, generation of the scheme in response to activation of the healthcare fraud attack alert may occur without notification to the human analyst. Thus, enabling a healthcare fraud attack alert may be a shortcut for generating a scheme.

The priority alert window 930 may display entities that will receive a "high" and/or "important" priority level when those entities share fraud data and/or schemes with the particular entity viewing/operating the user interface 902. For example, in the priority alert window 930, entities 1, 5, and 10 are selected for priority alerts. In this example, the priority alert window 930 comprises an "edit" option to add and/or remove entities for priority alerts.

The fraud attacks window 940 may display the healthcare fraud attacks that have been identified for the particular entity operating/viewing the user interface 902. For example, the attacks window 940 displays healthcare fraud attacks 1, 2, and 3. The attacks window 940 may be populated automatically by the healthcare fraud sharing system and/or by the entity. In the example attacks window 940, there is a "create" option, which may allow the human analyst to add a healthcare fraud attack. The attacks window 940 may have a "share" option, which may allow the human analyst to select one or more healthcare fraud attacks for sharing through the healthcare fraud sharing system, and may have options to share the healthcare fraud data, modified healthcare fraud data, and/or a scheme regarding the healthcare fraud attack (possibly in connection with other healthcare fraud attacks). The attacks window 940 may also have an "edit" option, which may allow the human analyst to edit and/or modify a healthcare fraud attack. For example, a healthcare fraud attack 1 may be an unnecessary procedures and/or tests attack, and healthcare fraud attack 1 may be edited to add and/or remove procedures, diagnoses, accounts, and/or membership identifiers. Alternatively, such updates may be performed automatically by the entity and/or the healthcare fraud sharing system based on predetermined schemes for modifying fraud data.

The schemes window 950 may display the schemes from an entity. The schemes window 950 may be operated similar to the attacks window 940, except the schemes window may be configured to display, create, and/or modify schemes instead of healthcare fraud attacks. For example, scheme 1 may be a stolen identifier scheme with a threshold for two pharmacies per day, and the example scheme may be edited to a threshold of three pharmacies per day. Schemes may be selected to display further information regarding the schemes, such as the various schemes of the scheme, one or more entities having fraud data on which the scheme was based, other entities with which the scheme has been shared (and/or is available for sharing), and/or entities that have actually implemented healthcare fraud measures in view of the scheme, for example.

Referring to Figure 9B, the example user interface 960 comprises a search box 964, an object display area 966, and/or a menu bar 962. A human analyst by typing and/or entering data into the search box 964 may load, lookup, and/or retrieve one or more objects. The user interface 960 may display healthcare fraud data, schemes, and/or other healthcare fraud information in clusters, which may correspond to the systems, methods, and/or techniques disclosed in the Ontology and/or Cluster references.

For example, by typing the name of a type of healthcare fraud attack, such as "upcoding," a fraud data object 968 may be displayed in the object display area 966. The other objects 970 (including objects 970A, 970B, and/or 970C) may be displayed automatically and/or after user interaction by the human analyst with the person object 410. The objects 970 may correspond to related healthcare fraud attacks, resources of the entity that have been attacked, and/or any other data object in the healthcare fraud sharing system. The one or more links 972 (including links 972A, 972B, and/or 972C) may display relationships between the fraud data object 968 and related objects 970. In other examples, an object search may be performed by entering a pharmacy identifier, membership identifier, drug code, and/or procedure code.

In addition to visually searching and/or showing data objects and/or relationships between data objects, the user interface 960 may allow various other manipulations. For example, data objects may be inspected (e.g., by viewing properties and/or associated data of the data objects), filtered (e.g., narrowing the universe of objects into sets and subsets by properties or relationships), and statistically aggregated (e.g., numerically summarized based on summarization criteria), among other operations and visualizations.

### Implementation Mechanisms

The various computing device(s) discussed herein, such as the entities 110 and/or healthcare fraud sharing system 100, are generally controlled and coordinated by operating system software, such as, but not limited to, iOS, Android, Chrome OS, Windows XP, Windows Vista, Windows 7, Windows 8, Windows Server, Windows CE, Unix, Linux, SunOS, Solaris, Macintosh OS X, VxWorks, or other compatible operating systems. In other embodiments, the computing devices may be controlled by a proprietary operating system. Conventional operating systems control and schedule computer processes for execution, perform memory management, provide file system, networking, I/O services, and provide a user interface functionality, such as a graphical user interface ("GUI"), among other things. The healthcare fraud sharing system 100 may be hosted and/or executed on one or more computing devices with one or more hardware processors and with any of the previously mentioned operating system software.

Figure 10 is a block diagram that illustrates example components of the healthcare fraud sharing system 100. While Figure 10 refers to the healthcare fraud sharing system 100, any of the other computing devices discussed herein may have some or all of the same or similar components.

The healthcare fraud sharing system 100 may execute software, e.g., standalone software applications, applications within browsers, network applications, etc., whether by the particular application, the operating system, or otherwise. Any of the systems discussed herein may be performed by the healthcare fraud sharing system 100 and/or a similar computing system having some or all of the components discussed with reference to Figure 10.

The healthcare fraud sharing system 100 includes a bus 1002 or other communication mechanism for communicating information, and a hardware processor, or multiple processors, 1004 coupled with bus 1002 for processing information. Hardware processor(s) 1004 may be, for example, one or more general purpose microprocessors.

The healthcare fraud sharing system 100 also includes a main memory 1006, such as a random access memory (RAM), cache and/or other dynamic storage devices, coupled to bus 1002 for storing information and instructions to be executed by processor(s) 1004. Main memory 1006 also may be used for storing temporary variables or other intermediate information during execution of instructions to be executed by processor(s) 1004. Such instructions, when stored in storage media accessible to processor(s) 1004, render the healthcare fraud sharing system 100 into a special-purpose machine that is customized to perform the operations specified in the instructions. Such instructions, as executed by hardware processors, may implement the methods and systems described herein for sharing healthcare fraud information.

The healthcare fraud sharing system 100 further includes a read only memory (ROM) 1008 or other static storage device coupled to bus 1002 for storing static information and instructions for processor(s) 1004. A storage device 1010, such as a magnetic disk, optical disk, or USB thumb drive (Flash drive), etc., is provided and coupled to bus 1002 for storing information and instructions. The scheme unit 530, attack modification unit 540, scheme data store 532 and/or fraud data store 542 of Figure 5 may be stored on the main memory 1006 and/or the storage device 1010.

In some embodiments, the scheme data store 532 of Figure 5 is a file system, relational database such as, but not limited to, MySql, Oracle, Sybase, or DB2, and/or a distributed in memory caching system such as, but not limited to, Memcache, Memcached, or Java Caching System. The fraud data store 542 of Figure 5 may be a similar file system, relational database and/or distributed in memory caching system as the scheme data store 532.

The healthcare fraud sharing system 100 may be coupled via bus 1002 to a display 1012, such as a cathode ray tube (CRT) or LCD display or touch screen, for displaying information to a computer user. An input device 1014 is coupled to bus 1002 for communicating information and command selections to processor 504. One type of input device 1014 is a keyboard including alphanumeric and other keys. Another type of input device 1014 is a touch screen. Another type of user input device is cursor control 1016, such as a mouse, a trackball, a touch screen, or cursor direction keys for communicating direction information and command selections to processor 1004 and for controlling cursor movement on display 1012. This input device may have two degrees of freedom in two axes, a first axis (e.g., x) and a second axis (e.g., y), that allows the device to specify positions in a plane. In some embodiments, the same direction information and command selections as cursor control may be implemented via receiving touches on a touch screen without a cursor.

The healthcare fraud sharing system 100 may include a user interface unit to implement a GUI, for example, Figure 9A and/or Figure 9B, which may be stored in a mass storage device as executable software codes that are executed by the computing device(s). This and other units may include, by way of example, components, such as software components, object-oriented software components, class components and task components, processes, functions, attributes, procedures, subroutines, segments of program code, drivers, firmware, microcode, circuitry, data, databases, data structures, tables, arrays, and variables.

In general, the word "instructions," as used herein, refers to logic embodied in hardware or firmware, or to a collection of software units, possibly having entry and exit points, written in a programming language, such as, but not limited to, Java, Lua, C, C++, or C#. A software unit may be compiled and linked into an executable program, installed in a dynamic link library, or may be written in an interpreted programming language such as, but not limited to, BASIC, Perl, or Python. It will be appreciated that software units may be callable from other units or from themselves, and/or may be invoked in response to detected events or interrupts. Software units configured for execution on computing devices by their hardware processor(s) may be provided on a computer readable medium, such as a compact disc, digital video disc, flash drive, magnetic disc, or any other tangible medium, or as a digital download (and may be originally stored in a compressed or installable format that requires installation, decompression or decryption prior to execution). Such software code may be stored, partially or fully, on a memory device of the executing computing device, for execution by the computing device. Software instructions may be embedded in firmware, such as an EPROM. It will be further appreciated that hardware modules may be comprised of connected logic units, such as gates and flip-flops, and/or may be comprised of programmable units, such as programmable gate arrays or processors. Generally, the instructions described herein refer to logical modules that may be combined with other modules or divided into sub-modules despite their physical organization or storage.

The healthcare fraud sharing system 100, or components of it, such as the scheme unit 530 and/or the attack modification unit 540 of Figure 5, may be programmed, via executable code instructions, in a programming language.

The term "non-transitory media," and similar terms, as used herein refers to any media that store data and/or instructions that cause a machine to operate in a specific fashion. Such non-transitory media may comprise non-volatile media and/or volatile media. Non-volatile media includes, for example, optical or magnetic disks, such as storage device 1010. Volatile media includes dynamic memory, such as main memory 1006. Common forms of non-transitory media include, for example, a floppy disk, a flexible disk, hard disk, solid state drive, magnetic tape, or any other magnetic data storage medium, a CD-ROM, any other optical data storage medium, any physical medium with patterns of holes, a RAM, a PROM, and EPROM, a FLASH-EPROM, NVRAM, any other memory chip or cartridge, and networked versions of the same.

Non-transitory media is distinct from but may be used in conjunction with transmission media. Transmission media participates in transferring information between nontransitory media. For example, transmission media includes coaxial cables, copper wire and fiber optics, including the wires that comprise bus 1002. Transmission media can also take the form of acoustic or light waves, such as those generated during radio-wave and infra-red data communications.

Various forms of media may be involved in carrying one or more sequences of one or more instructions to processor(s) 1004 for execution. For example, the instructions may initially be carried on a magnetic disk or solid state drive of a remote computer. The remote computer may load the instructions into its dynamic memory and send the instructions over a telephone or cable line using a modem. A modem local to the healthcare fraud sharing system 100 may receive the data on the telephone or cable line and use an infra-red transmitter to convert the data to an infra-red signal. An infra-red detector can receive the data carried in the infra-red signal and appropriate circuitry can place the data on bus 1002. Bus 1002 carries the data to main memory 1006, from which the processor(s) 1004 retrieves and executes the instructions. The instructions received by main memory 1006 may retrieve and execute the instructions. The instructions received by main memory 1006 may optionally be stored on storage device 1010 either before or after execution by processor(s) 1004.

The healthcare fraud sharing system 100 also includes a communication interface 1018 coupled to bus 1002. Communication interface 1018 provides a two-way data communication coupling to a network link 1020 that is connected to a local network 1022. For example, communication interface 1018 may be an integrated services digital network (ISDN) card, cable modem, satellite modem, or a modem to provide a data communication connection to a corresponding type of telephone line. As another example, communication interface 1018 may be a local area network (LAN) card to provide a data communication connection to a compatible LAN (or WAN component to be communicated with a WAN). Wireless links may also be implemented. In any such implementation, communication interface 1018 sends and receives electrical, electromagnetic or optical signals that carry digital data streams representing various types of information.

Network link 1020 typically provides data communication through one or more networks to other data devices. For example, network link 1020 may provide a connection through local network 1022 to a host computer 1024 or to data equipment operated by an Internet Service Provider (ISP) 1026. ISP 1026 in turn provides data communication services through the world wide packet data communication network now commonly referred to as the "Internet" 1028. Local network 1022 and Internet 1028 both use electrical, electromagnetic or optical signals that carry digital data streams. The signals through the various networks and the signals on network link 1020 and through communication interface 1018, which carry the digital data to and from the healthcare fraud sharing system 100, are example forms of transmission media.

The healthcare fraud sharing system 100 can send messages and receive data, including program code, through the network(s), network link 1020 and communication interface 1018. In the Internet example, a server 1030 might transmit a requested code for an application program through Internet 1028, ISP 1026, local network 1022 and communication interface 1018.

The received code may be executed by processor(s) 1004 as it is received, and/or stored in storage device 1010, or other non-volatile storage for later execution.

Each of the processes, methods, and algorithms described in the preceding sections may be embodied in, and fully or partially automated by, code instructions executed by one or more computer systems or computer processors comprising computer hardware. The processes and algorithms may be implemented partially or wholly in application-specific circuitry.

The various features and processes described above may be used independently of one another, or may be combined in various ways. All possible combinations and subcombinations are intended to fall within the scope of this disclosure. In addition, certain method or process blocks may be omitted in some implementations. The methods and processes described herein are also not limited to any particular sequence, and the blocks or states relating thereto can be performed in other sequences that are appropriate. For example, described blocks or states may be performed in an order other than that specifically disclosed, or multiple blocks or states may be combined in a single block or state. The example blocks or states may be performed in serial, in parallel, or in some other manner. Blocks or states may be added to or removed from the disclosed example embodiments. The example systems and components described herein may be configured differently than described. For example, elements may be added to, removed from, or rearranged compared to the disclosed example embodiments.

Conditional language, such as, among others, "can," "could," "might," or "may," unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without user input or prompting, whether these features, elements and/or steps are included or are to be performed in any particular embodiment.

Any process descriptions, elements, or blocks in the flow diagrams described herein and/or depicted in the attached figures should be understood as potentially representing units, segments, or portions of code which include one or more executable instructions for implementing specific logical functions or steps in the process. Alternate implementations are included within the scope of the embodiments described herein in which elements or functions may be deleted, executed out of order from that shown or discussed, including substantially concurrently or in reverse order, depending on the functionality involved, as would be understood by those skilled in the art.

It should be emphasized that many variations and modifications may be made to the above-described embodiments, the elements of which are to be understood as being among other acceptable examples. All such modifications and variations are intended to be included herein within the scope of this disclosure. The foregoing description details certain embodiments of the invention. It will be appreciated, however, that no matter how detailed the foregoing appears in text, the invention can be practiced in many ways. As is also stated above, it should be noted that the use of particular terminology when describing certain features or aspects of the invention should not be taken to imply that the terminology is being re-defined herein to be restricted to including any specific characteristics of the features or aspects of the invention with which that terminology is associated. The scope of the invention should therefore be construed in accordance with the appended claims and any equivalents thereof.

Insofar as embodiments of the invention described above are implementable, at least in part, using a software-controlled programmable processing device such as a general purpose processor or special-purposes processor, digital signal processor, microprocessor, or other processing device, data processing apparatus or computer system it will be appreciated that a computer program for configuring a programmable device, apparatus or system to implement the foregoing described methods, apparatus and system is envisaged as an aspect of the present invention. The computer program may be embodied as any suitable type of code, such as source code, object code, compiled code, interpreted code, executable code, static code, dynamic code, and the like. The instructions may be implemented using any suitable high-level, low-level, object-oriented, visual, compiled and/or interpreted programming language, such as C, C++, Java, BASIC, Perl, Matlab, Pascal, Visual BASIC, JAVA, ActiveX, assembly language, machine code, and so forth. A skilled person would readily understand that term "computer" in its most general sense encompasses programmable devices such as referred to above, and data processing apparatus and computer systems in whatever format they may arise, for example, desktop personal computer, laptop personal computer, tablet, smart phone or other computing device.

Suitably, the computer program is stored on a carrier medium in machine readable form, for example the carrier medium may comprise memory, removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD) subscriber identity module, tape, cassette solid-state memory. The computer program may be supplied from a remote source embodied in the communications medium such as an electronic signal, radio frequency carrier wave or optical carrier waves. Such carrier media are also envisaged as aspects of the present invention. Further aspects and embodiments of the invention are enumerated in the following numbered clauses.
1. Non-transitory computer storage comprising instructions for causing one or more computing devices to share healthcare fraud information by:
   receiving a plurality of healthcare related data from one or more open source data sources;
   receiving a scheme from a first entity of a plurality of entities, wherein the scheme is configured to be executable by one or more entities to recognize one or more healthcare fraud attacks based at least in part on healthcare related data from at least one open source data source;
   transmitting the scheme to one or more entities, wherein said transmitting of the scheme to one or more entities is in accordance with sharing rules established by the one or more entities.
2. The non-transitory computer storage of clause 1, further comprising:
   redacting portions of the scheme from various entities based on preferences of respective entities, such that the redacted portions are not detectable in the scheme.
3. The non-transitory computer storage of clause 1 or 2, further comprising:
   access weighting data associated with respective open source data sources providing the healthcare related data, wherein the weightings are indicative of a reliability of healthcare related data from respective open source data sources.
4. The non-transitory computer storage of any of clauses 1 to 3, wherein one or more open source data sources comprise at least one of provider exclusion lists, provider identifiers, physical mail data source, social security death master data source, or durable medical equipment supplier data source.
5. The non-transitory computer storage of any of clauses 1 to 4, wherein the scheme is further configured recognize one or more healthcare fraud attacks based at least in part on a match between a provider address and a physical drop box mail address.
6. The non-transitory computer storage of any of clauses 1 to 5, further comprising:
   generating one or more lists for display to an analyst, wherein the one or more lists correspond to the recognized one or more healthcare fraud attacks.
7. A computer-implemented method for sharing healthcare fraud information comprising:
   receiving a plurality of healthcare fraud data from one or more entities, the healthcare fraud data comprising information regarding one or more healthcare fraud attacks detected by respective entities;
   receiving a scheme, the scheme based at least in part on the plurality of healthcare fraud attack information from the one or more entities, wherein the scheme is configured to be executable by a plurality of the entities to recognize one or more healthcare fraud attacks; and
   transmitting the scheme to one or more entities.
8. The computer-implemented method of clause 7, further comprising:
   accessing weighting data associated with respective entities providing the healthcare fraud data, wherein the weightings are indicative of a reliability of healthcare fraud data from respective entities.
9. The computer-implemented method of clause 7 or 8, further comprising:
   generating one or more lists for display to an analyst, wherein the one or more lists correspond to the recognized one or more healthcare fraud attacks.
10. The computer-implemented method of any of clauses 7 to 9, wherein the scheme is further configured to recognize one or more healthcare fraud attacks based at least in part on benchmark data.
11. The computer-implemented method of clauses 10, wherein the scheme is further configured to recognize one or more healthcare fraud attacks based at least in part on a suspected fraud perpetrator being an outlier among the benchmark data.
12. The computer-implemented method of clause 11, wherein the benchmark data comprises prescription data for one or more drugs prescribed from respective providers.
13. A computer program comprising the instructions of the non-transitory computer storage medium of any of clauses 1 to 6, or comprising instructions executable by a computer apparatus to implement the method of clauses 7 to 12.

## Claims

1. A computer-implemented method for sharing healthcare fraud information comprising:
receiving first healthcare fraud data from a first entity, the first healthcare fraud data comprising information regarding one or more first healthcare fraud attacks detected by the first entity;
receiving second healthcare fraud data from a second entity, the second healthcare fraud data comprising information regarding one or more second healthcare fraud attacks detected by the second entity;
receiving a healthcare fraud detection scheme, the healthcare fraud detection scheme based at least in part on the first healthcare fraud data and the second healthcare fraud data;
applying the healthcare fraud detection scheme at a third entity to one or more healthcare data objects associated with a particular healthcare member of the third entity, wherein the healthcare fraud detection scheme comprises instructions to identify at least one of the following conditions:
a number of visits by the particular healthcare member at a healthcare location is above a visit threshold,
respective visit times of the particular healthcare member at two or more healthcare locations occurred within a predetermined time threshold,
a quantity of a particular procedure or product associated with the healthcare member is above a predetermined usage threshold within a time window,
a quantity of products associated with the healthcare member that are each included in an identified group of products is above a predetermined grouping threshold,
a recorded procedure and diagnosis combination for the healthcare member is not within a set of approved procedure and diagnosis combinations, or
a prescribing healthcare professional has prescribed a prescription to the particular healthcare member that is not included in a set of preapproved prescriptions associated with a profession type or category of the prescribing healthcare professional; and
in response to applying the healthcare fraud detection scheme, providing an alert to the third entity indicating a third potential or actual healthcare fraud attack; optionally, the method further comprising automatically generating a healthcare fraud detection scheme responsive to detecting the one or more first healthcare fraud attacks and/or the one or more second healthcare fraud attacks.

2. The computer-implemented method of claim 1, further comprising:
accessing weighting data associated with the first entity, wherein the weightings are indicative of a reliability of the first healthcare fraud data from the first entity; and/or further comprising:
generating one or more lists for display to an analyst, wherein the one or more lists correspond to the third potential or actual healthcare fraud attack.

3. The computer-implemented method of claim 1 or 2, wherein the healthcare fraud detection scheme further comprises instructions to recognize one or more healthcare fraud attacks based at least in part on benchmark data.

4. The computer-implemented method of claim 3, wherein the healthcare fraud detection scheme further comprises instructions to recognize one or more healthcare fraud attacks based at least in part on a suspected fraud perpetrator being an outlier among the benchmark data.

5. The computer-implemented method of any preceding claim, wherein each object of the one or more healthcare data objects associated with the particular healthcare member comprises a data container for properties, and wherein each property of the properties comprises one or more property types corresponding to one or more property values, and wherein the one or more healthcare data objects are retrieved from a non-transitory computer-readable storage medium.

6. A system for sharing healthcare fraud information, the system comprising:
one or more computing devices programmed, via executable code instructions, to implement:
a fraud data unit configured to receive a plurality of healthcare fraud data from one or more entities, the healthcare fraud data comprising information regarding one or more healthcare fraud attacks detected by respective entities;
a scheme unit configured to receive a scheme from a first entity of a plurality of entities, the scheme based at least in part on healthcare fraud data associated with the first entity, wherein the scheme is configured to be executable by one or more entities to recognize one or more healthcare fraud attacks;
a fraud data modification unit configured to redact portions of the healthcare fraud data from various entities such that the redacted portions are not detectable in the healthcare fraud data, and wherein the fraud data modification unit is further configured to redact portions of the scheme such that the redacted portions are not detectable in the scheme; and
a distribution unit configured to share the healthcare fraud data with multiple entities, and wherein the distribution unit is further configured to share the scheme with multiple entities.

7. The system of claim 6, wherein the fraud data modification unit is further configured to:
access weighting data associated with respective entities providing the healthcare fraud data, wherein the weightings are indicative of a reliability of healthcare fraud data from respective entities; and/or
wherein the one or more computing devices is further programmed, via executable code instructions, to implement:
a user interface unit configured to generate one or more clusters for display to an analyst, wherein the one or more clusters correspond to the recognized one or more healthcare fraud attacks.

8. The system of claim 6, wherein the plurality of healthcare fraud data include first healthcare fraud data from a first entity and second healthcare fraud data from a second entity, and the scheme is shared with a third entity.

9. The system of claim 6, wherein the scheme is further configured to recognize one or more healthcare fraud attacks based at least in part on benchmark data.

10. The system of claim 9, wherein the scheme is further configured to recognize one or more healthcare fraud attacks based at least in part on a suspected fraud perpetrator being an outlier among the benchmark data.

11. The system of claim 8, wherein sharing rules of the first entity indicate that the healthcare fraud data is shared with only a particular one or more entities; and/or
wherein an identity of the first entity is not included in the scheme based at least in part on preferences established by the first entity.

12. Non-transitory computer storage comprising instructions for causing one or more computing devices to share healthcare fraud information by:
receiving a plurality of healthcare related data from one or more open source data sources;
receiving a scheme from a first entity of a plurality of entities, wherein the scheme is configured to be executable by one or more entities to recognize one or more healthcare fraud attacks based at least in part on healthcare related data from at least one open source data source;
transmitting the scheme to one or more entities, wherein said transmitting of the scheme to one or more entities is in accordance with sharing rules established by the one or more entities.

13. The non-transitory computer storage of claim 12, further comprising:
redacting portions of the scheme from various entities based on preferences of respective entities, such that the redacted portions are not detectable in the scheme.

14. A computer-implemented method for sharing healthcare fraud information comprising:
receiving a plurality of healthcare fraud data from one or more entities, the healthcare fraud data comprising information regarding one or more healthcare fraud attacks detected by respective entities;
receiving a scheme, the scheme based at least in part on the plurality of healthcare fraud attack information from the one or more entities, wherein the scheme is configured to be executable by a plurality of the entities to recognize one or more healthcare fraud attacks; and
transmitting the scheme to one or more entities.

15. The computer-implemented method of claim 14, further comprising:
accessing weighting data associated with respective entities providing the healthcare fraud data, wherein the weightings are indicative of a reliability of healthcare fraud data from respective entities.

16. A computer program comprising the instructions of the non-transitory computer storage medium of any of claims 12 or 13, or comprising instructions executable by a computer apparatus to implement the method of claim 14 or 15.
